# FASCICULE DE BREVET EUROPEEN

(11) **EP 0 235 046 B1**
(45) Date de publication et mention de la délivrance du brevet: **16.06.1993**
(21) Numéro de dépôt: 87400434.4
(22) Date de dépôt: 27.02.1987
(51) Int. Cl.: C12Q 1/68, C07H 21/00, C12P 19/34, C12N 15/10, G01N 33/50, A61K 35/52, G01N 33/53, G01N 33/531, A01K 67/02

(54) **Sondes d'ADN spécifique du génome mâle des ruminants, leur préparation et utilisation**
DNS-Probe, spezifisch für das männliche Genom der Wiederkäuer, ihre Herstellung und Verwendung
DNA probe specific for the male genomes of ruminants, their preparation and use

(30) Priorité: 28.02.1986 FR 8602811; 09.09.1986 FR 8612616
(43) Date de publication de la demande: 02.09.1987
(73) Titulaire: INSTITUT NATIONAL DE LA RECHERCHE AGRONOMIQUE, Etablissement public dit:, F-75341 Paris Cédex 07 (FR); INSTITUT PASTEUR, 75724 Paris Cédex 15 (FR); COMMISSARIAT A L'ENERGIE ATOMIQUE Etablissement de Caractère Scientifique Technique et Industriel, 75015 Paris (FR)
(72) Inventeur: Bishop, Colin, F-92200 Neuilly S/Seine (FR); Cotinot, Corinne, F-94800 Villejuif (FR); Fellous, Marc, F-75005 Paris (FR); Kirszenbaum, Marek, F-78350 Jouy-en-Josas (FR); Vaiman, Marcel, F-75013 Paris (FR)
(74) Mandataire: Orès, Bernard

(56) Documents cités:
- WO-A-84/01265
- WO-A-86/00342
- WO-A-86/07095
- FR-A- 2 476 321
- NUCLEIC ACIDS RESEARCH, vol. 12, no. 6, mars 1984, IRL Press LTD, Oxford, GB; M.FROMMER et al., pp. 2887-2900
- NATURE, vol. 303, no. 5920, 30 juin 1983; C.E.BISHOP et al., pp. 831-832
- NATURE, vol. 307, no. 5947, 12 janvier 1984; G.GUELLAEN et al., pp. 172-173
- PROCEEDINGS OF THE NATL. ACADEMY OF SCIENCES USA, vol. 80, décembre 1983; pp. 7571-7575
- CELL, vol. 37, no. 1, mai 1984; E.E.LAMAR et al., pp. 171-177
- THE LANCET, i (8376), janvier 1984; YUN-FAI LAU et al., pp. 14-16
- CHEMICAL ABSTRACTS, vol. 105, no. 9, septembre 1986, Columbus, OH (US); Y.NISHIOKA et al., p. 165, no. 73564h
- CHEMICAL ABSTRACTS, vol. 103, no. 19, 11 novembre 1985, Columbus, OH (US); Y.F.LAU, p. 213, no. 155214s
- BIOLOGICAL ABSTRACTS, vol. 81, no. 6, juin 1986, Biological Abstracts Inc.; BURNS et al., no. 52843
- BIOLOGICAL ABSRACTS, vol. 30, 1986, Philadelphia, PA (US); L.L.McMAMEE, no. 99019
- NUCLEIC ACIDS RESEARCH, vol. 13, no. 5, mars 1985, IRL Press Ltd, Oxford, GB; A.CHOLLET et al., pp. 1529-1541

## Description

La présente invention est relative à la préparation de sondes moléculaires d'ADN spécifique utiles pour la détermination du sexe - ou sexage - d'embryons ou de foetus de ruminants, notamment de la sous-famille des bovinés, et en particulier du genre Bos, présentant des séquences d'ADN spécifique du sexe mâle.

Le sexage des embryons et des foetus présente un grand intérêt à plusieurs égards. La détermination du sexe d'embryons humains permet une détection précoce de maladies génétiques graves liées au sexe de l'embryon. La détermination du sexe d'embryons d'animaux d'élevage (ou sexage) présente quant à elle un grand intérêt économique.

Comme on le sait, le sexe (mâle ou femelle) est déterminé par les chromosomes sexuels. Chez les mammifères la présence d'un chromosome Y est suffisante pour produire un phénotype mâle et il semble que les gènes déterminant le sexe présents sur le chromosome Y se comportent comme un trait génétique dominant.

La première démarche adoptée pour la détermination précoce du sexe des embryons humains a donc consisté en une analyse chromosomique de cellules de liquide amniotique mises en culture. Cependant comme ce type d'analyse dure deux à trois semaines, il est apparu nécessaire de pourvoir à d'autres méthodes de détermination précoce du sexe d'embryons, susceptibles de donner des résultats plus rapides.

Il a été établi que la totalité du chromosome Y n'est pas impliquée dans la différenciation sexuelle. Il a donc paru logique d'analyser les séquences présentes sur le chromosome Y et la première démarche dans ce sens a consisté à isoler et cloner l'ADN du chromosome Y.

C'est ainsi que KUNKEL et Al. (notamment dans BIOCHEM vol. 18, n° 15 p. 3343-3353, 1979) ont isolé des fragments de 3,4 kilobases (kb) d'ADN de mâles humains par coupure à l'aide d'endonucléases de restriction telles que HaeIII, EcoRI ou EcoRII et ont montré que l'ADN répétitif spécifique du chromosome Y (ADN Y-it) est présent dans ces molécules de 3,4 kb. Cependant si les travaux de KUNKEL et Al. donnent des indications précieuses sur l'organisation de deux types de séquences répétitives, respectivement spécifique et non spécifique du chromosome Y, dans chaque molécule de 3,4 kb, ils ne sont pas utilisables directement dans le sexage.

Les travaux de BISHOP et Al. (NATURE, vol. 303,30 Juin 1983, p. 831-832) ont visé à construire une librairie partielle du chromosome Y en utilisant un hybride de cellules somatiques contenant uniquement le chromosome Y humain sur une base murine, obtenu par fusion de lymphocytes humains mâles avec la lignée cellulaire murine RAG et contenant un nombre modal de quatre chromosomes Y humains par cellule; ils ont extrait de l'hybride de l'ADN de poids moléculaire élevé, et ont réalisé une digestion partielle à l'aide de l'enzyme de restriction MboI et ont cloné les fragments de 35-50 kb dans des bactéries Escherichia coli en utilisant le cosmide pJB8. Pour différencier les clones contenant des inserts d'ADN humains (dérivés d'Y) de ceux contenant de l'ADN murin, les colonies ont été testées à basse densité avec de l'ADN humain répétitif ³²P-marqué, dans le but de ne détecter que les séquences répétitives et de créer la banque visée. Les Auteurs ont sélectionné parmi les clones recombinés des séquences d'ADN non-répétitif dérivé de Y afin d'étudier l'organisation du chromosome Y humain au niveau moléculaire. Ces travaux dont l'intérêt scientifique est déterminant, ne sont pas, eux non plus, directement utilisables pour le sexage des embryons de mammifères d'élevage.

Plusieurs méthodes de détermination précoce du sexe d'embryons humains ont été proposées sur la base des travaux de KUNKEL et Al., c'est-à-dire en utilisant des séquences d'ADN spécifique du chromosome Y. En particulier LAU et Al. (THE LANCET, 7 Janvier 1984, p. 14-16) ont proposé un test de détermination du sexe chez l'embryon humain à l'aide d'une sonde isolée à partir de la séquence répétitive humaine de 3,4 kb dérivée de l'hétérochromatine du chromosome Y (méthode de KUNKEL et Al.). Cette sonde a ensuite été hybridée à de l'ADN isolé d'un petit nombre de cellules provenant de 0,2 ml de liquide amniotique. Le taux d'hybridation est 1 000 fois plus grand à l'égard de l'ADN génomique mâle (isolé de cellules de liquide amniotique) qu'à l'égard de l'ADN génomique femelle. La méthode proposée par GOSDEN et Al. (THE LANCET, 10 Mars 1984, p. 540-541) qui est la méthode des "DOT BLOT" utilise une sonde spécifique du chromosome Y, dénommée pHY2,1 précédemment décrit par ces Auteurs. Cette sonde s'hybride à de l'ADN provenant d'une biopsie de villosités choriales d'un embryon humain mâle.

LAMAR et PALMER (CELL, vol. 37, Mai 1984, p. 171-177) ont cherché à développer une méthode générale de clonage de l'ADN du chromosome Y chez la souris. La méthode proposée par ces Auteurs consiste à fragmenter l'ADN de foie de souris femelles par sonication et à digérer complètement de l'ADN de souris mâles par MbOI. Un échantillon de l'ADN femelle fragmenté et l'ADN mâle digéré sont mélangés dans une proportion de 100:1. Le mélange est dénaturé et réassocié à un Cot=1320 dans un tampon approprié : dans ces conditions, 95% de l'ADN est sous forme double-brin. Ceux-ci sont constitués d'ADN femelle homogène, de double-brins hétérogènes mâle-femelle correspondant aux séquences communes aux deux sexes et enfin de duplex spécifiques mâles. Les double-brins sont isolés par chromatographie sur colonne d'hydroxylapatite. Ils sont recombinés dans le plasmide pBR 322. Les plasmides recombinants sont utilisés pour transformer des bactéries Escherichia coli HB 101 compétentes. Environ 100 000 clones (Amp^{r}-Tet^{s}) ont été testés, parmi lesquels 3 000 contiennent de l'ADN présumé spécifique du chromosome Y. Trois de ces sondes se sont révélées être spécifiques de la souris mâle, ce qui tendrait à démontrer une spécificité d'espèce des sondes moléculaires d'ADN mâle.

Par ailleurs, plusieurs techniques de tri des spermatozoïdes en vue de leur sexage ont été proposées :
- des méthodes dites physiques de séparation des spermatozoïdes portant respectivement des chromosomes X et Y, par électrophorèse (SHISHITO et al., Int. J. Fertil, 20, 13-16, 1976), gel-filtration, passage dans un gradient d'albumine (ERICSSON et al., Nature, vol. 246, 421-424, 1973), centrifugation et plus particulièrement par centrifugation dans un gradient de densité en utilisant de Percoll (Fertility and Sterility, vol. 40, n° 5, Nov. 1983), par ultracentrifugation sur un gradient de densité de saccharose, etc ...
- des méthodes de fractionnement du sperme en fractions riches en spermatozoïdes portant le chromosome Y et/ou en fractions riches en spermatozoïdes portant le chromosome X, en tirant parti des différences de mobilité des spermatozoïdes porteurs du chromosome Y par rapport à ceux porteurs du chromosome X, dans différents milieux (gradients d'albumine par exemple) (Brevet US 4 009 260); ainsi que des méthodes de séparation des spermatozoïdes propres à provoquer une séparation en fonction de la densité, en tirant parti du fait que la densité du chromosome Y est inférieure à celle du chromosome X (Brevet US 3 894 529), ces méthodes comprenant l'application de forces hydrostatiques (Brevets US 4 092 229 et US 4 155 831), la centrifugation dans un gradient d'un milieu approprié tel que Percoll par exemple. On peut également séparer une population de spermatozöides en fractions différentes, du fait de la charge électrique liée aux sexes différentes, à l'aide d'un matériau porteur d'une charge électrostatique (Brevet US 4 083 957). Au nombre de ces méthodes de séparation on peut également ranger les méthodes de tri cellulaire par cytométrie de flux, c'est-à-dire par déviation et séparation des cellules identifiées par leur quantité d'ADN, par un champ électrique, suivie de l'étude des cellules triées par exemple par fixation d'autres ligands marqués, par microscopie optique ou électronique, par des tests fonctionnels in vitro, etc ... (Application in Cell Biology, chap. 25, 471-483, 1980).
- des méthodes immunologiques dont certaines utilisent des anticorps sélectivement réactifs avec des spermatozoïdes portant soit le chromosome X, soit le chromosome Y, qui sont agglutinés et inactivés, libérant ainsi une fraction de spermatozoïdes portant de façon homogène le chromosome de type opposé à celui qui a réagi avec l'anticorps susdit, la fraction fixée à l'anticorps pouvant également être libérée de celui-ci, le cas échéant, notamment dans le cas où l'anticorps est fixé à une phase solide constituée par un matériau immunoabsorbant (Brevets US 4 191 749, US 4 085 205 et US 3 687 806).

Les publications dans lesquelles les méthodes précitées sont également décrites sont les suivantes :
- SHILLING E. In : "Sex-ratio et birth prospects for Control." (C.A. KIDDY and H.D. HAFS. Eds) Am. Soc. Animal. Sci.pp. 76-84 (1971)
- MEISTRICH M.L. J. Cell. Physiol., 299-312 (1972)
- RHODE W. et al., J. Repro. Fertil. 42, 587-591 (1975)
- STEENO O. et al., Andrologia, 7, 95-97 (1975)
- BHATTACHARYA B.C. et al., Ind. J. Exp. Biol., 14, 610-611 (1976)
- SHASTRY P.R. et al., Nature, 269, 58-60 (1977)
- SHILLING E., THORMAHLEN D. Zuchthyg, 11, 113-121 (1976)
- ADIMOELJA A. et al., Andrologia 9, 289-292 (1977)
- BHATTACHARYA B.C. et al., Int. J. Fertil., 22, 30-35 (1977)
- SCHILLING E., THORMAHLEN D. Andrologia, 9, 74-78 (1977)
- PERTOFT H. et al., Anal. Biochem. 88, 271-282 (1978)
- SCHILLING E. et al., Andrologia 10, 215-217 (1978)
- QUINLIVAN W.L.G., Fertil. Steril., 34, 307-308 (1980)
- QUINLIVAN W.L.G. et al., Fertil. Steril, 37, 104-107 (1982)
- CORSON S.L. et al., Fertil. Steril, 40, 384-385 (1983)
- KANEKO S. et al., Fertil. Steril 40, 661-665 (1983)
- BEAL W.E. et al., J. Anim. Sci., 58, 1432-1436 (1984)
- KANEKO S. et al., Biomed. Res., 5, 187-194 (1984)
- UEDA K. et al., Dev. Growth Differ. 28, suppl. Abst. 79 (1986)
- ERICSSON R.J. et al. Nature, 246, 421-424 (1973)
- ROSS A. et al. Nature, 253, 354-355 (1975)
- SHISHITO et al., Int. J. Fertil., 20, 13-16 (1975)
- BROER K.H. et al., Andrologia, 9, 74-78 (1977)
- DMOWSKI W.P. et al., Fert. and Seril., 31, 52-57 (1979)
- QUINLIVAN W.L.G. et al., Fertil. Steril. 37, 104-107 (1982)
- KNAACK J. et al., Tierzucht 27, 156-159 (1973)
- LANG J.L., Chemtech. March, 189-192 (1973)
- SHIRAI M. et al., Tohoku. J. Exp. Med., 113 273-281 (174)
- MOORE H.D.M. et al., Reprod. Fertil., 44, 329-332 (1975)
- BATTACHARYA B.C. et al., Int. J. Fertil. 24, 256-259 (1979)
- HAMMERSTEDT R.H. et al., Arch. Biochem. Biophys. 194, 565-580 (1979)
- BENNETT D. and BOYSE E.A. Nature, 246, 3086309 (1973)
- BENNETT D. et al. Nature, 257, 237-238 (1975)
- SILVERS W.K. and WATCHEL S.S., Science, 195, 956-960 (1977)
- HANCOCK R.T.J., Biol, Reprod., 18, 510-515 (1978)
- SHALEV A., Diss Abst B, 40, 5538 (1980)
- HEDGE U.C. et al. J. Reprod. Immunol. 2, 351-357 (1981)
- WATCHET S.S. Theriogenology, 24, 419-429 (1984)
- GLEDHILL B.L. et al., In : Flow Cytometry and Sorting (M. Melamed, P. Mullaney and Mendelsohn, eds) John Wiley and Sons, Inc. N.Y. pp 471-484 (1979)
- MORUZZI J.F., Reprod. Fert, 57, 319-323 (1979)
- EVENSON D.P. et al., Science, 210, 1131-1133 (1980)
- EVENSON D.P. et al., Chromosoma, 78, 225-238 (1980)
- GROGAN W.M. et al., J. Histochem. Cytochem., 29, 738-746 (1981)
- STEEN H.B. et al., Cytometry, 2, 129 (1981)
- PINKEL D. et al., Science, 218, 904-905 (1982)
- PINKEL D et al., Cytometry, 3, 1-9 (1982)
- BENARON et al., Cytometry, 5 (1982)
- GARNER D.L. et al, Biol. Reprod., 28, 312-321 (1983)
- GLEDHILL B.L. et al., 10th Int. Congr. Anim. Reprod.
- PINKEL D. et al., J. Anim. Sci., 60, 1303-1307 (1985)
- JOHNSON L.A., et al., Gemete Res. in press (1986)
- JOHNSON L.A. and PINKEL D., Cytometry, 7, in press (1986)
- JOHNSON L.A., Beltsville Symp. Agric. Res., X, (in press) M. Nijhoff publ. (1986)
- SHETTLES L.B., Int. J. Gynaecol. Obstet, 8, 643-647 (1970)
- DOWNING D.C. and BLACK D.L. Fertil. Steril., 27, 1191-1193 (1976)
- MUEHLEIS P.M. and LONG S.Y. Fertil. Steril., 27, 1438-1445 (1976)
- PORSTMANN T. and SCHMECHTA H. Dermatol. Monatsschr., 165, 28-35 (1979)
- BARLOW P. and VOSA C.G., Nature, 226, 961-962 (1970)
- BEATTY R.A., Cytogenet cell Genet., 18i, 33-49 (1977)
- ERICSSON R.J. et al., In : Clinics in Andrology, vol. I, (J.C. Emperaire, A. Audebert, E.S.E. Hafez, eds). Martinus Nijhoff, The Hague pp 98-111 (1980)
- KIDDY C.A. and HAFS H.D., Sex ratio at birth. Prospect and control. Am. Soc. Anim. Sci. publ. (1971)
- COUROT M., Le choix du sexe est-il possible chez les mammifères ? In : "La Fécondation" pp 113-130, Masson publ. (1975)
- RINEHART W., Sec Preselection not yet practical. Population reports series I 2 pp 11 (1975)
- AMANN R.P. and SEIDEL G.E., Prospect for Sexing Mammalina Sperm, pp 288 Colorado Ass. Univ. Press. publ. (1982).

Les méthodes de tri des spermatozoïdes proposées dans l'Art antérieur ont pour inconvénient commun de présenter une marge d'incertitude relativement importante en ce qui concerne leur fiabilité : en effet, les procédés de tri ne donnent pas des fractions homogènes à 100% d'un chromosome ou de l'autre, en sorte que la qualité du tri ne peut être contrôlée dans la pratique qu'en procédant aux inséminations artificielles qui sont le but final visé (les fécondations in vitro ne donnant dans les faits qu'un faible rendement). D'autre part, pour savoir si la déviation du "sex ratio" a une valeur significative en liaison avec une fraction de spermatozoïdes isolée par l'une quelconque des méthodes de tri préconisées dans l'Art antérieur, il faut avoir procédé à un nombre important, statistiquement significatif, d'inséminations, ce qui peut s'avérer coûteux et long.

Il serait donc indispensable de pouvoir disposer d'un moyen propre à permettre un contrôle immédiat des fractions de spermatozoïdes obtenues par l'une quelconque des méthodes de tri proposées conformément à l'Art antérieur, afin de limiter ou même d'éliminer tout risque d'erreur au niveau de la réalisation des inséminations artificielles en utilisant les fractions triées en question.

La présente invention s'est donné pour but de pourvoir : à des sondes moléculaires d'ADN spécifique du génome mâle de ruminants, notamment de la sous-famille des bovinés, en particulier du genre Bos, présentant des séquences d'ADN spécifique du sexe mâle, à une méthode de détermination du sexe de ces animaux à l'aide des sondes susdites, qui répondent de façon satisfaisante aux besoins de la technique considérée en ce qu'elles permettent le sexage d'embryons de ces ruminants à partir d'un petit nombre de cellules embryonnaires, n'excédant pas 500 à 1 000, permettant la détermination du sexe des embryons à un stade précoce du développement, avec un taux de fiabilité de 100%.

La présente invention s'est également donné pour but de pourvoir à une méthode de contrôle immédiat de la proportion de spermatozoïdes portant le chromosome Y dans une fraction de population de spermatozoïdes séparés par une méthode de tri quelconque appropriée, en utilisant la sonde moléculaire conforme à la présente invention.

La présente invention a pour objet une sonde moléculaire d'ADN spécifique du génome mâle des ruminants, notamment de la sous-famille des bovinés, en particulier du genre Bos, caractérisée en ce qu'elle comprend 49 paires de bases dont la séquence 5'-3' en nucléotides est la suivante : 
5'                                                    3'
ATCAGTGCAGGGACCGAGATGTGCTCCAAGGAGTGTTTATCGGCTGCTT,
ainsi que son complémentaire, ou un fragment de ladite séquence comportant au moins 11 nucléotides consécutifs ou tout autre fragment présentant au moins 70% d'homologie avec ce dernier, laquelle sonde est désignée par la dénomination b.c.1.2, et présente un profil d'hybridation, déterminé par hybridation de ladite sonde avec de l'ADN génomique mâle digéré EcoRI qui fait apparaître la présence d'une bande de l'ordre de 7 kb qui est spécifique du génome mâle du genre BOS.

La sonde moléculaire conforme à la présente invention est répétée au moins 2000 fois dans le génome mâle du genre BOS.

Sa séquence en nucléotides a été déterminée par la méthode de MAXAM et GILBERT [METHODS ENZYMOL., 65, 499 (1977)].

L'hybridation de la sonde b.c.1.2. avec de l'ADN génomique mâle digéré par EcoRI révèle la présence d'une bande de 7 kilobases (kb) absente de l'ADN génomique femelle.

La présente invention a également pour objet des procédés de préparation d'une sonde moléculaire d'ADN spécifique du génome mâle de ruminants, notamment de la sous-famille des bovinés, en particulier du genre Bos.

Selon un aspect de la présente invention, un procédé de préparation d'une telle sonde moléculaire, qui réalise la réassociation de segments d'ADN génomique rendus simple brin, à partir d'un mélange d'ADN mâle et femelle dans lequel l'ADN femelle est en grand excès (de l'ordre de 100 parties pour 1 partie d'ADN mâle), est caractérisé en ce qu'il comprend : - une première étape au cours de laquelle de l'ADN génomique femelle desdits ruminants est coupé par sonication en fragments de 200 à 550 paires de bases (pb), puis mélangé dans une proportion de l'ordre de 100:1, à de l'ADN génomique mâle des mêmes ruminants, digéré par l'endonucléase SAU 3A pour libérer des fragments de restriction à extrémités cohésives, de taille comprise environ entre 4O et 1 650 pb; - une deuxième étape au cours de laquelle ledit mélange d'ADN, après avoir été extrait d'une manière connue en elle-même par un mélange phénol-chloroforme-alcool isoamylique et précipité par l'éthanol, est dénaturé à 100°C puis réassocié à 68°C pendant 22 heures dans un tampon contenant 2M(NH₄)₂SO₄, 50 mM phosphate de sodium (pH = 6,8) et 5 mM EDTA; - une troisième étape au cours de laquelle l'ADN double-brin réassocié est mélangé avec un plasmide tel que le plasmide pUC9 (Amp^{r}-Lac Z⁺) préalablement linéarisé par l'endonucléase BamHI et déphosphorylé par de la phosphatase alcaline, en présence de ligase T₄ qui réalise l'insertion de l'ADN dans le plasmide, par contact pendant 16 heures à 16°C; - une quatrième étape au cours de laquelle les plasmides recombinants obtenus lors de l'étape précédente, sont utilisés pour transformer des cellules d'Escherichia coli rendues hautement compétentes, les clones recombinants (Amp^{r}-Lac Z⁻) étant obtenus dans une proportion d'environ 200 clones pour 2 ml de suspension de cellules bactériennes.

Selon un mode de mise en oeuvre avantageux du procédé de préparation de la sonde moléculaire conforme à l'invention, les cellules bactériennes d'Escherichia coli sont rendues hautement compétentes, préalablement à l'insertion des plasmides recombinants, par traitement pendant dix minutes à 0°C par un milieu comprenant : 10 mM K-Mes (pH = 6,2), 100 mM RbCl₂, 45 mM MnCl₂, 3 mM [Co(NH₃)₆]Cl₃.

Selon un autre mode de mise en oeuvre avantageux du procédé conforme à la présente invention, les bactéries transformées sont étalées sur un milieu gélosé contenant de l'Ampicilline et du X-gal (ou 5-bromo-4-chloro-3-indolyl-β-D-galacto-pyranoside) à faible concentration dans du diméthylformamide, les colonies bactériennes isolées (Amp^{r}-Lac Z⁻) sont repiquées individuellement, puis amplifiées en vue de la préparation de minilysats, puis chaque plasmide isolé est digéré par l'endonucléase PvuII, marqué avec un ou plusieurs radioisotopes, et hybridé avec de l'ADN génomique provenant desdits ruminants et l'ADN de ces animaux est digéré par l'endonucléase EcoRI qui génère des fragments de restriction de taille variable.

Selon une disposition avantageuse de ce mode de mise en oeuvre, ces fragments sont séparés par migration électrophorétique sur gel d'agarose.

Selon un autre aspect du procédé de préparation d'une sonde moléculaire d'ADN spécifique du génome mâle des ruminants, notamment de la sous-famille des bovinés, en particulier du genre Bos, une telle sonde est obtenue par synthèse, le procédé de synthèse étant caractérisé en ce que la séquence en nucléotides que comprend ladite sonde est synthétisée par la technique de phosphoramidite en phase solide.

Selon un mode de mise en oeuvre préféré du procédé de synthèse de l'oligonucléotide qui entre dans la composition de la sonde moléculaire conforme à l'invention, la synthèse proprement dite est suivie d'une étape de purification qui consiste en une séparation de l'oligonucléotide d'une taille donnée recherché, associé à des oligonucléotides de taille intermédiaire, par électrophorèse sur un gel de polyacrylamide, puis repérage de la bande de l'oligonucléotide pur recherché, à l'aide d'une feuille d'aluminium recouverte de gel de silice, fluorescente à 254 nm, découpage de cette bande et élution de l'ADN dans une solution appropriée, puis récupération de l'ADN pur synthétisé.

Selon un autre mode de mise en oeuvre préféré du procédé de synthèse de la sonde moléculaire conforme à l'invention, cette dernière est marquée avec un ou plusieurs radioisotopes, après purification de l'oligonucléotide de synthèse.

La présente invention a également pour objet la mise en oeuvre des sondes moléculaires d'ADN spécifique du génome mâle de ruminants, notamment de la sous-famille des bovinés, en particulier du genre Bos, pour la détermination du sexe d'embryons et de foetus desdits ruminants, lequel procédé de détermination est caractérisé en ce qu'une sonde conforme à l'invention, hybridée à un filtre préhybridé de manière appropriée connue en elle-même, lorsqu'elle est réassociée à de l'ADN génomique desdits ruminants, mâle et femelle, préalablement transféré sur un filtre approprié, après avoir été isolé par électrophorèse sur gel d'agarose, s'hybride exclusivement à l'ADN génomique mâle, et cette hybridation est mise en évidence par autoradiographie et/ou par sonde non-radioactive, par la présence de signaux d'hybridation en forme de bandes [SOUTHERN (J. Mol. Biol. 98, 503 (1975)].

Selon un mode de réalisation avantageux de la méthode de sexage conforme à l'invention, l'ADN à reconnaître est contenu dans des lymphocytes des ruminants susdits.

Selon un autre mode de réalisation avantageux de la méthode de sexage conforme à l'invention appliquée au foetus, l'ADN à reconnaître est contenu dans des cellules issues du liquide amniotique ou allantoïdien ou de villosités choriales des ruminants susdits.

Conformément à l'invention, lesdites cellules ou analogues des ruminants susdits sont déposées sur membrane (Dot-Blot) ou sur un filtre approprié, sous forme de gouttes après avoir été soniquées.

Selon encore un autre mode de réalisation avantageux de la méthode de sexage conforme à l'invention, l'ADN à reconnaître est contenu dans le chromosome Y desdits ruminants, observé sur métaphases, auquel cas le sexage est réalisé par hybridation dite in situ.

Selon un autre mode de réalisation avantageux de la méthode de sexage conforme à l'invention, l'ADN à reconnaître est contenu dans des noyaux interphasiques des cellules desdits ruminants, auquel cas le sexage est réalisé par hybridation dite in situ.

Dans une hybridation in situ conforme à l'invention, une biopsie de 5 à 20 cellules provenant d'un embryon desdits ruminants est mise en contact avec la sonde moléculaire conforme à l'invention pour réaliser le marquage desdites cellules par hybridation avec ladite sonde marquée avec un ou plusieurs radioisotopes ou avec une sonde non-radioactive, puis l'hybridation est révélée d'après une technique cytoimmunochimique appropriée.

La présente invention a également pour objet un procédé de contrôle direct et immédiat de la proportion de spermatozoïdes portant le chromosome Y dans une population de spermatozoïdes de ruminants, notamment de la sous-famille des bovinés, en particulier du genre Bos, caractérisé en ce que les spermatozoïdes de ladite population sont mis en contact avec la sonde moléculaire conforme à l'invention, pour réaliser le marquage desdits spermatozoïdes par hybridation avec ladite sonde marquée d'une manière convenable, puis les spermatozoïdes hybridés sont révélés par une méthode cytoimmunochimique appropriée.

Selon un mode de mise en oeuvre avantageux du procédé de contrôle conforme à la présente invention, l'hybridation des spermatozoïdes avec la sonde moléculaire d'ADN spécifique est réalisée in situ directement avec les spermatozoïdes.

Selon un autre mode de mise en oeuvre avantageux du procédé de contrôle conforme à la présente invention, l'hybridation est réalisée entre la sonde moléculaire d'ADN spécifique et l'ADN isolé à partir de spermatozoïdes de ruminants du genre Bos.

Selon une disposition avantageuse du mode de mise en oeuvre dans lequel l'hybridation est réalisée in situ, les spermatozoïdes sont soumis préalablement à l'étape d'hybridation, à un processus de décondensation chimique qui comprend essentiellement trois étapes, à savoir : un choc hypotonique, une digestion protéolytique et une réduction des ponts disulfures.

Selon encore un autre mode de mise en oeuvre avantageux du procédé de contrôle conforme à la présente invention, la sonde moléculaire d'ADN spécifique mise en oeuvre est une sonde marquée avec un ou plusieurs radioisotopes, ou une sonde non-radioactive.

Le procédé de contrôle de la proportion de spermatozoïdes portant le chromosome Y, dans une population de spermatozoïdes, conforme à la présente invention, permet de mesurer la proportion de chromosomes Y présents dans cette population.

Conformément à la présente invention, on tire parti de cette possibilité de déterminer la quantité d'ADN Y spécifique dans les spermatozoïdes, à l'aide de la sonde d'ADN spécifique conforme à l'invention, pour développer un procédé de tri des spermatozoïdes X et Y selon leur teneur en ADN, dont les différentes étapes de séparation seront elles-mêmes contrôlées à l'aide de la sonde moléculaire spécifique du chromosome Y des ruminants conforme à l'invention.

La présente invention a en conséquence également pour objet un procédé de tri des spermatozoïdes porteurs du chromosome X et des spermatozoïdes porteurs du chromosome Y, dans une population de spermatozoïdes, qui est caractérisé par les étapes suivantes : - 1) marquage de l'ADN contenu dans les spermatozoïdes par un colorant vital spécifique de l'ADN (tel, par exemple, que le bromure d'éthidium, l'acridine, le colorant Hoechst 33342, le DAPI); - 2) tri par cytofluorométrie en deux fractions de spermatozoïdes X et Y en fonction de la quantité d'ADN qu'ils contiennent; - 3) contrôle de la qualité du tri opéré au cours de la 2ème étape du procédé, en mettant en oeuvre le procédé de contrôle de la proportion de spermatozoïdes porteurs du chromosome Y dans la fraction de spermatozoïdes Y séparée par tri, conforme à la présente invention; - 4) immunisation de mammifères rongeurs (souris ou rats) par les fractions contrôlées, éventuellement soumises à un nouveau tri à la suite du contrôle, en vue de la production d'anticorps monoclonaux ou polyclonaux; - 5) contrôle de la spécificité desdits anticorps, par des tests d'agglutination, de spermatotoxicité, d'immunofluorescence indirecte, de cytométrie de flux pour l'obtention de réactifs immunologiques spécifiques reconnaissant des déterminants antigéniques contrôlés par le chromosome du sexe recherché et exprimés à la surface des spermatozoïdes, aptes à être utilisés pour la séparation de spermatozoïdes vivants porteurs du chromosome X ou du chromosome Y; - 6) séparation des fractions de spermatozoïdes porteurs respectivement du chromosome X et du chromosome Y, dans une population de spermatozoïdes, à l'aide desdits réactifs immunologiques.

Selon un mode de mise en oeuvre avantageux du procédé de tri conforme à la présente invention, l'immunisation des mammifères rongeurs qui constitue la 4ème étape du procédé de tri est suivie de la collecte des anticorps polyclonaux résultant de ladite immunisation et de l'absorption de ceux-ci sur des spermatozoïdes de sexe opposé à celui ayant servi à l'immunisation, la spécificité des ces anticorps étant contrôlée sur des spermatozoïdes du sexe recherché, et lesdits anticorps étant ensuite éventuellement purifiés.

Selon un autre mode de mise en oeuvre avantageux du procédé de tri conforme à la présente invention, les spermatozoïdes X et Y séparés par cytofluorométrie sont utilisés pour préparer des anticorps monoclonaux spécifiques des chromosomes X et Y.

La présente invention a en outre pour objet les réactifs immunologiques spécifiques reconnaissant des déterminants antigéniques contrôlés par le chromosome X ou Y ayant servi à l'obtention desdits réactifs et exprimés à la surface des spermatozoïdes, caractérisés en ce qu'ils sont constitués par les anticorps obtenus en mettant en oeuvre le procédé ci-dessus, testés pour leur spécificité et éventuellement purifiés.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre de la présente invention.

Il doit être bien entendu, toutefois, que ces exemples sont donnés uniquement à titre d'illustration de l'objet de l'invention, dont ils ne constituent en aucune manière une limitation.

### EXEMPLE 1

### PREPARATION DE SONDES CONFORMES A L'INVENTION

La technique mise en oeuvre permet de cloner l'ADN présent dans le génome mâle et absent du génome femelle et a ainsi permis de construire une librairie d'ADN spécifique du bovin mâle.

### CONSTRUCTION DE LA LIBRAIRIE D'ADN SPECIFIQUE DU MALE

1 mg d'ADN génomique femelle provenant de lymphocytes périphériques est coupé par sonication en fragments de 200 à 550 paires de bases (pb). 10 µg d'ADN génomique mâle provenant de lymphocytes périphériques sont digérés par l'endonucléase SAU 3A (Boehringer), libérant ainsi des fragments de restriction à extrémités cohésives, de taille comprise environ entre 40 et 1 650 pb.

Ces deux types de fragments sont mélangés dans la proportion suivante : ADN femelle : ADN mâle = 100 : 1.

Après extraction par le mélange phénol-chloroforme-alcool isoamylique et précipitation à l'éthanol absolu, le mélange des deux ADN est dénaturé à 100°C pendant 10 mn puis réassocié à 68°C pendant 22 heures dans un tampon contenant 2M (NH₄)₂SO₄, 50 mM phosphate de sodium (pH = 6,8) et 5 mM EDTA. Un aliquot (1 µg) d'ADN double-brin réassocié est mélangé avec 100 ng de plasmide pUC9 (Amp^{r}-Lac Z⁺) préalablement linéarisé par l'endonucléase Bam Hl (Boehringer) et déphosphorylé par la phosphatase alcaline (CIP Boehringer). L'insertion de l'ADN bovin mâle dans le plasmide est effectuée avec de la ligase T₄ (Biolabs) à 16°C pendant 16 heures.

Les plasmides recombinants sont utilisés pour transformer 2 ml de bactéries Escherichia coli-JM83 rendues hautement compétentes par traitement par un milieu contenant : 10 mM K-Mes (pH = 6,2), 100 mM RbCl₂, 45 mM MnCl₂, 3 mM [Co(NH₃)₆]Cl₃ pendant 10 mn à 0°C. Les bactéries ainsi transformées sont étalées sur du milieu LB-Agar contenant 100 µg/ml d'Ampicilline et 2 µl/ml de X-gal (5-bromo-4-chloro-3-indolyl-β-D-galacto-pyranoside) à 2% dans le diméthylformamide. Approximativement 200 clones (Amp^{r}-Lac Z⁻) ont été obtenus.

### EXEMPLE 2

### ANALYSE DE LA LIBRAIRIE

Afin de rechercher la présence d'inserts d'ADN bovin mâle, chaque clone est repiqué individuellement et les plasmides sont isolés des bactéries par minilysat. Chaque plasmide isolé est digéré par l'endonucléase PvuII (Boehringer) et analysé par électrophorèse en gel d'agarose à 1% (30 volts - 16 h).

Cinquante clones, parmi les 200 analysés, contiennent de l'ADN bovin (insert).

La spécificité des inserts ainsi produits a été démontrée par hybridation avec de l'ADN génomique (lymphocytes) mâle et femelle digéré par l'endonucléase EcoRI (Boehringer) et transféré après électrophorèse sur du papier DBM ou des membranes HYBOND (AMERSHAM) selon la technique de SOUTHERN.

L'hybridation de la sonde b.c.1.2. avec de l'ADN génomique mâle digéré par EcoRI révèle la présence d'une bande de 7,0 kilobases (kb) absente de l'ADN génomique femelle. Ce résultat a été confirmé par l'étude de 12 animaux mâles et 13 femelles (p<10⁴).

### EXEMPLE 3

### PREPARATION DE L'ADN GENOMIQUE BOVIN

L'ADN bovin de mâle et de femelle est purifié à partir de 5x10⁷ lymphocytes périphériques. Les cellules sont incubées à 42°C pendant 18 heures sous agitation lente dans 10 ml de solution de lyse [10 mM Tris-HCl pH 7,5, 10 mM éthylènediaminetétraacétate-Na₂ (EDTA), 50 mM NaCl, 0,4% dodécyl sulfate de sodium plus 2 mg de protéinase K (Boehringer)]. 1 mg de ribonucléase A (Boehringer) dans 1 ml de 10 mM Tris-HCl pH 7,5; 1 mM EDTA, est ajouté en 1 heure à 37°C, suivi d'une seconde digestion par la Protéinase K (1 mg, 1 h à 42°C). Les lysats sont extraits 3 fois avec 15 ml d'un mélange composé de phénol : chloroforme : alcool isoamylique (3:1:0,01 v/v) saturé avec 0,2 M Tris-HCl pH 7,5. Les phases aqueuses sont lavées 3 fois avec une solution chloroforme-alcool isoamylique (1:0,04). L'ADN est précipité avec 2 volumes d'éthanol absolu en présence de 150 mM NaCl et lavé avec de l'éthanol à 80% dans l'eau.

### DIGESTION PAR LES ENDONUCLEASES DE RESTRICTION

Des échantillons de 15 µg d'ADN bovin sont digérés à 37°C pendant 18 heures avec l'endonucléase de restriction EcoRI (Boehringer) dans le tampon d'incubation contenant 100 mM Tris-HCl, 50 mM NaCl, 10 mM MgCl₂, pH 7,5. La digestion complète est obtenue avec un total de 6-10 unités par µg de l'ADN ajouté en 3 fois. Les fragments de l'ADN digérés sont soumis à une séparation électrophorétique en gel d'agarose de 0,7% dans du tampon 0,04 M Tris-CH₃COOH pH 8, 0,002 M EDTA. La calibrage du gel est obtenu en incluant un échantillon de l'ADN de phage-Lambda digéré par Hind III.

### TRANSFERT

Les fragments de l'ADN contenu dans le gel sont dépurinés pendant 15 mn à température ambiante dans du 0,25 M HCl, dénaturés en 0,5 M NaOH + 1,5 M NaCl, puis neutralisés par passages successifs dans du 1,5 M tampon phosphate pH 5,5 (3x15 mn) puis 0,15 M pH 5,5 (2x15 mn). Une membrane (Hybond-N Amersham ou Pall-Biodyne-A Flobio) en "Nylon" est ensuite placée sur le gel et par capillarité les fragments de l'ADN contenu dans le gel sont transférés sur la membrane. Après 16 heures de transfert en 20 x SSC, la membrane est lavée dans du 2 x SSC, séchée à l'air puis placée pour 2 heures dans un four à 80°C.

### MARQUAGE DE LA SONDE AU ³²P

100 à 200 ng de sonde b.c.1.2. sous un volume de 1 à 5 µl sont ajoutés au mélange réactionnel suivant : 50 µM de dATP (1 µl), 50 µM de dTTP (1 µl), 50 µM de dCTP 1 µl), 50 µCi de α-³²P-dGTP (5 µl), 2 µl de tampon de réaction contenant 500 mM Tris-HCl pH 7,5, 500 mM HgCl₂, 100 mM β-mercaptoéthanol. La réaction est effectuée à 16°C pendant 1 heure 30, puis arrêtée par addition de 200 µl de la solution contenant 10 mM Tris-HCl pH 7,5, 80 mM EDTA, 0,5% dodécyl sulfate de sodium.

La sonde radiomarquée est séparée des nucléotides par passage sur une colonne (10 cm x 1 cm) de Sephadex G100 (Pharmacia) et éluée par du tampon 10 mM Tris-HCl pH 7,5 1 mM EDTA. Les fractions d'élution contenant le plus de radioactivité sont ensuite poolées.

### PREHYBRIDATION ET HYBRIDATION

Les filtres (blots) sont préhybridés à 42°C pendant 4-10 heures avec un mélange contenant 50% v/v de formamide désionisée, 1 M NaCl, 0,2% de solution de Denhart, 2,5% de sulfate de dextran, 0,1% de dodécyl sulfate de sodium et 0,5 mg/ml d'ADN de sperme de saumon soniqué.

L'hybridation est réalisée dans le même mélange excepté le sulfate de dextran qui est à 5% et le NaCl à 0,5 M. La sonde radiomarquée au ³²P dénaturée à 100°C pendant 10 mn est ajoutée dans le mélange d'hybridation à 1 x 10⁶ cpm/ml, ce qui représente environ 10 ng de sonde par ml. L'hybridation est réalisée à 42°C pendant au moins 18 heures. Après l'hybridation, les filtres sont lavés une première fois à 65°C pendant 30 mn dans du tampon 2 x SSC (0,3 M NaCl et 0,03 M Citrate de sodium)+0,1% SDS puis encore 30 mn dans le même tampon dilué 10 fois (0,2 x SSC). Les filtres sont ensuite placés dans une casette équipée des intensificateurs "Cronex" (DUPONT), mise en contact avec un film KODAK XAR-5 pour l'autoradiographie pendant 6 à 24 heures à -70°C.

### DESHYBRIDATION

Elle est réalisée en lavant les filtres à 37°C pendant 30 mn avec du 0,4 M NaOH, puis dans une solution contenant : 0,2 M Tris-HCl pH 7,5, 0,1% (v/v) dodécyl sulfate de sodium, 0,1 x SSC.

### PREPARATION DES DOT-BLOT

### 1) Dot-Blot ADN génomique

L'ADN génomique des bovins mâle et femelle a été soniqué pendant 5 mn (Branson Sonifier B-15) puis dilué avec l'ADN du sperme de saumon soniqué dans des proportions telles que la quantité de l'ADN totale du dépôt soit de 2 µg. La gamme de concentration en ADN bovin était de 1 µg, 0,5 µg, 0,25 µg, 0,1 µg et 0,05 µg pour un dépôt de 1 µl.

Après le dépôt de l'ADN sur les membranes Pall-Biodyne A celles-ci sont traitées avec 0,5N NaOH + 1,5N NaCl pendant 5 mn puis neutralisées avec 1,5 M tampon phosphate pH 5,5 pendant 2 x 5 mn et 0,3 M tampon phosphate pH 5,5 pendant 5 mn encore. Les membranes sont par la suite séchées sur Whatman 3MM, puis l'ADN est fixé à 80°C pendant 2 heures. Les filtres prêts à l'emploi sont conservés dans la solution de préhybridation à 4°C.

### 2) Dot-Blot des cellules bovines

Les lymphocytes périphériques du bovin mâle et femelle ont été soniqués pendant 6 minutes puis traités par la protéinase K (Boehringer) 1 heure à 42°C. La gamme de dilution correspondant à : 1x10⁶, 5x10⁵, 1x10⁵, 5x10⁴, 1x10⁴ et 5x10³ cellules dans 2 µl. L'ADN du sperme de saumon soniqué est ajouté pour compléter à 2 µg de l'ADN total.

Après le dépôt de l'ADN sur membrane Pall-Biodyne A le traitement est identique à celui de dot-blot ADN génomique.

Hybridations des dot-blots et visualisation des signaux sont les mêmes que pour les blots en général.

### EXEMPLE 4

### SYNTHESE ET PURIFICATION DE L'OLIGONUCLEOTIDE DE SYNTHESE

La synthèse chimique de l'oligonucléotide correspondant à la séquence déterminée auparavant a été réalisée à l'aide d'un synthétiseur automatique "Applied Biosystems" selon la technique de phosphoramidite en phase solide.

La réaction étant efficace à 95-97% à chaque stade, il est donc nécessaire de purifier le produit final (sonde b.c.1.2. - 49 mer) des oligonucléotides intermédiaires. Environ 7 mg du mélange d'oligonucléotides dans 60 µl H₂O sont séparés par électrophorèse dans le gel de 20% polyacrylamide + 8 M urée à 700 V, 38mA pendant 3 heures. La bande correspondant au 49 mer est repérée à l'aide d'une feuille d'aluminium recouverte de gel de silice, fluorescente à 254 nm (Merck). Cette partie du gel est découpée puis l'ADN élué dans 1,5 ml de la solution 0,5 M CH₃COONH₄ + 5 mM EDTA sous agitation à 37°C pendant 16 heures.

Le surnageant est concentré à environ 300 µl et l'urée est éliminée par chromatographie sur une colonne 10 cm x 1 cm de Sephadex G-50 (Pharmacia) équilibré avec le tampon 10 mM Tris-HCl pH 7,5 + 1 mM EDTA. L'ADN ainsi isolé est lyophilisé et conservé à -20°C.

### MARQUAGE RADIOACTIF (³²P) DE LA SONDE b.c.1.2. DE SYNTHESE (Monobrin)

10 à 20 pM de la sonde b.c.1.2. dans un volume de 3 µl sont ajoutés à 23 µl du mélange réactionnel composé de : 100 mM dithiothréitol (2,5 µl), 10 mM spermidine (2,5 µl), tampon d'incubation 500 mM Tris-HCl pH 7,5 + 100 mM MgCl₂ (2,5 µl), 100 µCi (γ-³²P) adénosine triphosphate-ATP 3 000Ci/mmol (Amersham) et 20 U T4 polynucléotide kinase (Boehringer). Après incubation de 30 mn à 37°C la réaction est arrêtée avec 2 µl 400 mM EDTA et l'oligonucléotide marqué est séparé de l'ATP par chromatographie sur colonne avec Sephadex G-50.

### EXEMPLE 5

### SEQUENCAGE

### - PREPARATION DU PLASMIDE b.c.1.2. EN VUE DU SEQUENCAGE DE SES 2 BRINS

### a) Brin inférieur (sens 3ʹ → 5ʹ)

Trente microgrammes de plasmide b.c.1.2. sont digérés par l'endonucléase Hind III pendant 3 heures à 37°C. L'enzyme est éliminée par extraction avec le mélange phénol : chloroforme : alcool isoamylique (3:1:0,01 v/v). L'ADN est ensuite précipité dans 1 volume d'isopropanol en présence de 0,3M CH₃COONa, puis lavé dans de l'éthanol 80% dans l'eau.

L'ADN du plasmide linéarisé est marqué en 3ʹ par l'enzyme terminale transférase avec du didéoxyadénosine triphosphate ³²P (AS = 3 000 Ci/mM) selon la technique du Kit n° N 4020 de chez AMERSHAM.

| | |
|---|---|
| DNA du plasmide | 40 µl |
| ddATP 125 µCi) | 13 µl |
| Tampon Amersham x 10 | 10 µl |
| Eau distillée | 95 µl |
| Solution enzymatique AMERSHAM | 5 µl |
| Incubation 1 heure à 37°C. | |

Le plasmide marqué est ensuite séparé du nucléotide par passage sur Sephadex G75 puis digéré par l'endonucléase EcoRI qui génère 2 fragments de taille 2,6 et 0,1 kb respectivement. Le fragment de 0,1 kb contenant l'insert b.c.1.2. est isolé par électrophorèse en gel d'agarose 2%. Il est ensuite purifié par passage sur laine de verre et extraction avec le mélange phénol-chloroforme-alcool isoamylique.

Le fragment purifié est séquencé selon la méthode de MAXAM et GILBERT modifiée par la Société N.E.N.

L'analyse des fragments à séquencer est effectuée par électrophorèse en gel de 0,2 mm à 5%, 8% et 12% de polyacrylamide sur une cuve Macrophore LKB [GAROFF et ANSORGE, Analytical Biochemistry 115 (450-457) 1980]. Selon les concentrations les temps de migration ont été : 6 h-7 h et 2 h.

Les gels sont ensuite fixés avec de l'acide acétique 10% pendant 10 mn, lavés à l'eau, puis séchés à température ambiante.

L'autoradiographie du gel est effectuée sur film URNO HS 90 pendant des temps d'exposition de 24, 48 et 72 heures.

### b) Brin supérieur (sens 5ʹ → 3ʹ)

Mêmes techniques, sauf que la première enzyme de digestion est EcoRI et la deuxième PVUII.

La détermination de la séquence en nucléotides de la sonde b.c.1.2., obtenue à partir de lymphocytes périphériques de mammifères du genre Bos, qui est composée des 49 paires de basis suivantes : 
5'                                                    3'
ATCAGTGCAGGGACCGAGATGTGCTCCAAGGAGTGTTTATCGGCTGCTT
a permis de synthétiser la sonde conforme à l'invention comme décrit à l'Exemple 4 ci-dessus.

La sonde b.c.1.2. conforme à l'invention est d'une grande sensibilité puisque des tests réalisés sur membranes PALL BIODYNE ont montré que 50 ng d'ADN bovin sont une quantité suffisante pour obtenir un signal positif.

### EXEMPLE 6

### DETERMINATION DE LA BANDE ADN SPECIFIQUE DU CHROMOSOME Y DANS UNE POPULATION DONNEE DE SPERMATOZOIDES

### A. Préparation d'ADN à partir de sperme bovin

Chaque paillette d'environ 15 à 20 x 10⁶ spermatozoïdes congelés est immédiatement plongée dans un bain-marie à 37°C pendant environ 30 secondes. Le contenu de chaque paillette est ensuite dilué dans 1 à 2 ml de PBS stérile. Chaque tube est centrifugé à 500 g pendant 10 mn. Le surnageant est éliminé et le culot repris dans 1 à 2 ml de PBS stérile. Ce lavage est répété 4 fois. Après la dernière centrifugation, le culot de spermatozoïdes est remis en suspension dans 10 ml de tampon de lyse contenant 0,01 M Tris-HCl pH 8,0; 0,1 M NaCl; 0,01 M EDTA; 1% SDS; 2% β-mercaptoéthanol. Ce tampon est préchauffé à 50°C. Après 30 minutes d'incubation à 50°C, 2 mg de protéinase K (concentration finale 200 µg/ml) sont ajoutés dans chaque tube et l'incubation est poursuivie pendant 2 heures sous agitation lente à 50°C.

Le mélange est ensuite extrait pendant 15 minutes à température ambiante avec 15 ml d'une solution de 50% de phénol saturé en Tris 0,2 M pH 7,5 et 50% de chloroforme : alcool isoamylique (24:1). La phase inférieure organique est éliminée et l'extraction est répétée 2 autres fois.

La phase aqueuse est ensuite extraite 2 à 3 fois pendant 15 minutes à température ambiante avec un mélange chloroforme : alcool isoamylique (24:1). Après la dernière extraction la phase aqueuse est centrifugée à 2 000 g pendant 30 minutes pour éliminer totalement le chloroforme et les protéines précipitées. Le surnageant clarifié est alors mélangé à 2 1/2 volumes d'éthanol absolu préalablement refroidi pendant 15 minutes à - 20°C. L'ADN précipité, il est récupéré à l'aide d'une pipette stérile, lavé pendant 5 mm dans de l'éthanol 70%. L'excès d'alcool est évaporé et l'ADN dissous dans du tampon stérile 0,001 M Tris-HCl pH 8,0 et 0,0001 M EDTA.

### B. Digestion par les endonucléases de restriction

Des échantillons de 15 µg d'ADN de spermatozoïdes bovins sont digérés à 37°C pendant 18 heures avec l'endonucléase de restriction EcoRI (Boehringer) dans le tampon d'incubation contenant 100 mM Tris-HCl, 50 mM NaCl, 10 mM MgCl₂, pH 7,5. La digestion complète est obtenue avec un total de 6-10 unités par µg de l'ADN ajouté en 3 fois. Les fragments de l'ADN digérés sont soumis à une séparation électrophorétique en gel d'agarose de 0,7% dans du tampon 0,04 M Tris-CH₃COOH pH 8, 0,002 M EDTA. Le calibrage du gel est obtenu en incluant un échantillon de l'ADN de phage-Lambda digéré par Hind III.

### Transfert

Les fragments de l'ADN contenu dans le gel sont dépurinés pendant 15 mn à température ambiante dans du 0,25 M HCl, dénaturés en 0,5 M NaOH + 1,5 M NaCl, puis neutralisés par passages successifs dans du 1,5 M tampon phosphate pH 5,5 (3 x 15 mn) puis 0,15 M pH 5,5 (2 x 15 mn). Une membrane (Hybond-N Amersham ou Pall-Biodyne-A Flobio) en "Nylon" est ensuite placée sur le gel et par capillarité les fragments de l'ADN contenu dans le gel sont transférés sur la membrane. Après 16 heures de transfert en 20 x SSC, la membrane est lavée dans du 2 x SSC, séchée à l'air puis placée pour 2 heures dans un four à 80°C.

### Marquage de la sonde au ³²P

100 à 200 ng de sonde b.c.1.2. sous un volume de 1 à 5 µl sont ajoutés au mélange réactionnel suivant : 50 µM de dATP (1 µl), 50 µM de dTTP (1 µl), 50 µM de dCTP (1 µl), 50 µCi de α-³²P-dGTP (5 µl), 2 µl de tampon de réaction contenant 500 mM Tris-HCl pH 7,5, 500 mM MgCl₂, 100 mM β-mercaptoéthanol. La réaction est effectuée à 16°C pendant 1 heure 30, puis arrêtée par addition de 200 µl de la solution contenant 10 mM Tris-HCl pH 7,5, 80 mM EDTA, 0,5% dodécyl sulfate de sodium.

La sonde radiomarquée est séparée des nucléotides par passage sur une colonne (10 cm x 1 cm) de Sephadex G100 (Pharmacia) et éluée par du tampon 10 mM Tris-HCl pH 7,5, 1 mM EDTA. Les fractions d'élution contenant le plus de radioactivité sont ensuite poolées.

### C. Préhybridation et hybridation

Les filtres (blots) sont préhybridés à 42°C pendant 4-10 heures avec un mélange contenant 50% v/v de formamide désionisée, 1 M NaCl, 0,2% de solution de Denhart, 2,5% de sulfate de dextran, 0,1% de dodécyl sulfate de sodium et 0,5 mg/ml d'ADN de sperme de saumon soniqué.

L'hybridation est réalisée dans le même mélange excepté le sulfate de dextran qui est à 5% et le NaCl à 0,5 M. La sonde radiomarquée au ³²P dénaturée à 100°C pendant 10 mn est ajoutée dans le mélange d'hybridation à 1 x 10⁶ cpm/ml, ce qui représente environ 10 ng de sonde par ml. L'hybridation est réalisée à 42°C pendant au moins 18 heures. Après l'hybridation, les filtres sont lavés une première fois à 65°C pendant 30 mn dans du tampon 2 x SSC (0,3 M NaCl et 0,03 M citrate de sodium) + 0,1% SDS puis encore 30 mn dans le même tampon dilué 10 fois (0,2 x SSC). Les filtres sont ensuite placés dans une cassette équipée des intensificateurs "Cronex" (DUPONT), mise en contact avec un film KODAK XAR-5 pour l'autoradiographie pendant 6 à 24 heures à - 70°C.

### D. Résultats

L'ADN de spermatozoïdes bovins digéré par l'enzyme de restriction EcoRI puis hybridé avec la sonde b.c.1.2. radiomarquée, présente un profil d'hybridation analogue à celui obtenu avec les autres types cellulaires bovins testés par les Inventeurs.

### EXEMPLE 7

### DETERMINATION DU SEXE DES EMBRYONS BOVINS PAR HYBRIDATION IN SITU AVEC UNE SONDE D'ADN BIOTINYLEE (NON-RADIOACTIVE)

### A. Préparation des cellules

Les cellules utilisées proviennent d'embryons bovins agés de 7 à 8 jours de gestation. Après avoir été disséquées, les cellules sont déposées sur une lame et incubées pendant 10 à 15 minutes dans une solution de fixateur tel que le mélange alcool-acide acétique (3 : 1 v/v) puis séchées à l'air. Les lames sont utilisées immédiatement ou, en cas de sexage différé, conservées à 4°C à l'abri des poussières.

### B. Biotinylation de la sonde

On utilise un fragment du plasmide pUC9 contenant l'insert b.c.1.2., l'ensemble faisant 350 pb. Le fragment de 350 pb est obtenu par digestion par l'enzyme de restriction PvuII, purifié par électrophorèse sur gel d'agarose et sorti du gel au moyen de l'absorption sur membrane DEAE NA45 (Schleicher and Schuell).

La biotinylation de la sonde s'effectue par nick-translation, en utilisant les constituants suivants, sous un volume final de 0,050 ml.

| | |
|---|---|
| dATP dGTP dCTP | 0,2 mM chacun |
| BRL DNA Polymerase I | 0,4 unité/ul |
| DNase I | 40 pg/ul |
| DNA à marquer | 1 µg |
| d UTP biotiné | 0,4 mM |

On laisse incuber 90 minutes à 15°C, on arrête la réaction avec 300 mM Na₂ EDTA + 5% SDS (Protocole BRL).

Après marquage, la sonde est purifiée par gel-filtration sur colonne de Sephadex G-100. Les différentes fractions collectées sont analysées séparément à l'aide du système streptavidine-phosphatase alcaline (DNA Detection System BRL réf. B239 SA).

Les fractions positives sont mélangées et l'activité du pool est mesurée par comparaison avec une quantité connue de DNA biotinylé.

### C. Technique d'hybridation sur lames

Les lames sont incubées avec 90 à 100 µl de liquide d'hybridation et recouvertes d'un film plastique (pour éviter l'évaporation).

Après dénaturation pendant 10 mn dans un four à 100°C, les lames sont mises en chambre humide et incubées à 42°C pendant 16 heures.

La composition finale du liquide d'hybridation est :
4 x SSC
1 mM EDTA
25 mM Tris-HCl pH 7,3
1 x Denhardt
10% Dextran
50% Formamide déionisé
Sonde : 80 ng/ml de milieu d'hybridation
(1 x SSC = 0,15 M NaCl, 0,015 M citrate de Na)
(1 x Denhardt = 0,02% BSA, ou 0,02% lait écrémé en poudre;
0,02% polyvinylpyrrolidone
0,02% Ficoll).

Après avoir ôté le film plastique, les lames sont lavées successivement en solution 2 x SSC (30 minutes à température ambiante), puis en 0,1 x SSC (30 minutes à 42°C), 2 x SSC (15 minutes à température ambiante) et enfin en PBS pH 7,4 additionné de 0,1% v/v de Triton x - 100 et 0,5% (p/v) de lait écrémé en poudre (Regilait).

### D. Révélation

Les lames encore humides après avoir ôté l'excédent de fluide sont alors incubées 1 heure à 37°C avec un anticorps de chèvre antibiotine ou un anticorps de lapin antibiotine. Cet anticorps est vendu purifié par chromatographie d'affinité (Vector Laboratories ref. SP-3000) et est utilisé dans nos tests dilué au 1:350 dans du PBS pH 7,4 + 0,1% (v/v) Triton x - 100 + 0,5% (p/u) lait écrémé en poudre.

Après un lavage de 15 minutes à température ambiante dans PBS pH 7,4 + 0,1% (v/v) Triton x - 100 + 0,5% (p/v) lait écrémé en poudre, les lames sont alors incubées 1 heure à 37°C avec un anticorps conventionnel de lapin anti-chèvre ou de chèvre anti-lapin couplé à la peroxydase (Biosys ref. BI 2403) dilué 1:40 dans PBS pH 7,4 + Triton 0,1% + lait écrémé en poudre 0,5%.

Les lames sont ensuite lavées deux fois 15 minutes dans PBS pH 7,4 contenant cette fois 0,1% (v/v) de Tween 20 et finalement dans du PBS pH 7,4 pendant 5 minutes.

On procède à une incubation de 5 à 8 minutes à température ambiante avec une solution de diaminobenzidine (DAB SIGMA ref. D 8001) à 0,5 mg/ml dans PBS pH 7,4 contenant 0,01% de H₂O₂.

Le précipité formé par l'action de la peroxydase sur la DAB est amplifié par précipitation successive de sels d'or et d'argent selon le procédé décrit par BURNS et al (Journal Clinical Pathology 1985, 35, 1085-1092) :

Dans un premier temps on dépose 50 à 100 µl par lame d'une solution 2,5 mM de chloroaurate de Na (NaAuCl₄ BDH ref. 30125 2R) et on incube 5 mn à température ambiante. Après un lavage de 5 mn dans de l'eau distillée, les lames sont incubées 5 mn à température ambiante avec 50 à 100 µl d'une solution 0,1 M de sulfure de Na (Na₂S, SIGMA ref. S 2006), rincées 5 mn à l'eau distillée et incubées 4 à 8 minutes avec 100 à 300 µl de réactif à l'argent.

La composition du réactif à l'argent est la suivante :

| | | | |
|---|---|---|---|
| A | Carbonate de Na | 0,24 M | (SIGMA ref. S 4132) |
| B1 | Nitrate d'Ammonium | 13 mM | (SIGMA ref. A 9642) |
| B2 | Nitrate d'Argent | 6 mM | (BDH ref. 303873N) |
| B3 | Ac. Dodecatunstosilicique | 1,5 mM | (BDH ref. 305453R) |
| B4 | Formaldéhyde 37% | 0,6 ul/ml | (SIGMA ref. F 1635) |

Le réactif à l'argent est préparé en mélangeant successivement et sous agitation les solutions B1 - B2 - B3 - B4. Le mélange obtenu est additionné 1:1 à la solution A et le réactif ainsi préparé est utilisé immédiatement.

Après le traitement au réactif à l'argent, les lames sont lavées à l'H₂O distillée pendant 15 minutes, puis dans l'acide acétique 1% (v/v) 2 fois 15 minutes et enfin colorées pendant 1 minute en Pyronin Y (SIGMA ref. P 7017) 1% en H₂O, rincées pendant quelques secondes à l'eau distillée, séchées à l'air chaud et montées en DPX.

### E. Résultats des spermatozoïdes

Après hybridation in situ la présence d'un marquage spécifique est détectable sur les spermatozoïdes. Le bruit de fond est négligeable. L'augmentation du volume de la tête des spermatozoïdes témoigne d'une décondensation de ceux-ci.

Après hybridation in situ la présence du marquage est observée à l'aide d'un microscope optique.

Le marquage spécifique est détectable sur environ 80 à 100% des cellules mâles et est totalement absent des cellules femelles. Il se présente sous forme d'un précipité brun-noir au niveau des noyaux des cellules mâles.

### EXEMPLE 8

### CONTROLE DE LA QUALITE DU TRI D'UNE POPULATION DE SPERMATOZOIDES BOVINS AVEC UNE SONDE D'ADN SPECIFIQUE DU SEXE MALE

### A. Préparation des cellules

Les cellules utilisées sont des spermatozoïdes de taureau congelés et stockés dans l'azote liquide. On décongèle une paillette, soit environ 13 000 000 de spermatozoïdes vivants. Les paillettes sont réchauffées pendant 30 secondes à 37°C puis le sperme dilué dans 1 ml de PBS stérile.

On procède à une série de centrifugations dans le but d'éliminer au maximum le milieu de congélation. La suspension des spermatozoïdes en PBS est d'abord centrifugée pendant 10 mn à 500 g, le surnageant aspiré et les spermatozoïdes repris en 1 ml de 0,11 M citrate de sodium. On centrifuge 10 mn à 500 g. On aspire le surnageant, on reprend en 1 ml de 0,11 M citrate de sodium + 5% DMSO, on centrifuge 10 mn à 500 g. On aspire le surnageant, on resuspend dans 1 ml de 0,11 M citrate de sodium + 15% DMSO. On centrifuge 10 mn à 500 g. On aspire le surnageant, on resuspend en 1 ml de 0,11 M citrate de sodium + 50% DMSO. On centrifuge 10 mn à 500 g. On aspire le surnageant; on resuspend dans 1 ml de 0,11 M citrate de sodium dans du Tris-HCl 0,1 M pH 7,4. On centrifuge 10 mn à 500 g. On aspire le surnageant et on resuspend dans 250 µl de ce même tampon.

A ce stade, les cellules sont soit déposées sur lame (30 - 40 µl de suspension de spermatozoïdes par lame) et traitées par hybridation in situ avec une sonde biotinylée, soit déposées sur filtre et hybridées selon la technique des dot-blots avec une sonde marquée à l'aide de nucléotides radioactifs.

### B. Prétraitement des lames pour l'hybridation in situ

Après le dépôt des spermatozoïdes, les lames sont séchées à l'air et la décondensation des cellules est la suivante :
1) on procède à un choc hypotonique à l'aide d'une solution de chlorure de potassium 0,08 M pendant 5 mn à température ambiante. Les spermatozoïdes sont ensuite fixés dans un mélange méthanol : acide acétique (3:1) pendant 30 mn. Afin d'éliminer l'activité de la peroxydase endogène, les lames sont traitées au méthanol + H₂O₂ 3,3% pendant 30 mn, puis rincées dans l'éthanol absolu 10 mn, xylène 10 mn, éthanol absolu 10 mn.
2) après séchage à l'air, une solution de 40 U de papaïne (activée avec 2 mM de sulfure de sodium dans du Tris 0,1 M pH 8,6) (ou de la protéinase K ou de la trypsine) est déposée sur chaque lame.
3) après 15 mn d'incubation à 37°C, les lames sont brièvement rincées dans du PBS puis incubées pendant 15 mn à température ambiante dans une solution de Tris 0,1 M pH 8,6 contenant 1% de DMSO ou de SDS et 40 mM d'un réducteur des ponts disulfure tel que dithiothreitol, β-mercaptoéthanol ou analogues. Les lames sont ensuite lavées 3 fois 5 mn dans du PBS puis séchées à l'air.
   A ce stade les lames sont prêtes pour la dénaturation et l'hybridation in situ.

### C. Technique d'hybridation sur lames

Les lames sont incubées avec 90 à 100 µl de liquide d'hybridation et recouvertes d'un film plastique (pour éviter l'évaporation).

Après dénaturation pendant 10 mn dans un four à 100°C, les lames sont mises en chambre humide et incubées à 42°C pendant 16 heures.

La composition finale du liquide d'hybridation est :
4 x SSC
1 mM EDTA
25 mM Tris-HCl pH 7,3
1 x Denhardt
10% Dextran
50% Formamide déionisée
Sonde : 80 ng/ml de milieu d'hybridation
(1 x SSC = 0,15 m NaCl, 0,015 M citrate de Na)
(1 x Denhardt = 0,02% BSA, ou 0,02% lait écrémé en poudre;
0,02% polyvinylpyrrolidone
0,02% Ficoll).

Après avoir ôté le film plastique, les lames sont lavées successivement en solution 2 x SSC (30 minutes à température ambiante), puis en 0,1 x SSC (30 minutes à 42°C), 2 x SSC (15 minutes à température ambiante) et enfin en PBS pH 7,4 additionné de 0,1% v/v de Triton x - 100 et 0,5% (p/v) de lait écrémé en poudre (Regilait).

### D. Révélation

Les lames encore humides après avoir ôté l'excédent de fluide sont alors inclubées 1 heure à 37°C avec un anticorps de chèvre antibiotine ou un anticorps de lapin antibiotine. Cet anticorps est vendu purifié par chromatographie d'affinité (Vector Laboratories ref. SP-3000) et est utilisé dans nos tests dilué au 1:350 dans du PBS pH 7,4 + 0,1% (v/v) Triton x - 100 + 0,5% (p/u) lait écrémé en poudre.

Après un lavage de 15 minutes à température ambiante dans PBS pH 7,4 + 0,1% (v/v) Triton x - 100 + 0,5 (p/v) lait écrémé en poudre, les lames sont alors incubées 1 heure à 37°C avec un anticorps conventionnel de lapin anti-chèvre ou de chèvre anti-lapin couplé à la peroxydase (Biosys ref. BI 2403) dilué 1:40 dans PBS pH 7,4 + Triton 0,1% + lait écrémé en poudre 0,5%.

Les lames sont ensuite lavés deux fois 15 minutes dans PBS pH 7,4 contenant cette fois 0,1% (v/v) de Tween 20 et finalement dans du PBS pH 7,4 pendant 5 minutes.

On procède à une incubation de 5 à 8 minutes à température ambiante avec une solution de diaminobenzidine (DAB SIGMA ref. D 8001) à 0,5 mg/ml dans PBS pH 7,4 contenant 0,01% de H₂O₂.

Le précipité formé par l'action de la peroxydase sur la DAB est amplifié par précipitation successive de sels d'or et d'argent selon le procédé décrit par BURNS et al (Journal Clinical Pathology (1985, 35, 1085-1092) :

Dans un premier temps on dépose 50 à 100 µl par lame d'une solution 2,5 mM de chloroaurate de Na (NaAuCl₄ BDH ref. 30125 2R) et on incube 5 mn à température ambiante. Après un lavage de 5 mn dans de l'eau distillée, les lames sont incubées 5 mn à température ambiante avec 50 à 100 µl d'une solution 0,1 M de sulfure de Na (Na₂S, SIGMA ref. S 2006), rincées 5 mn à l'eau distillée et incubées 4 à 8 minutes avec 100 à 300 µl de réactif à l'argent.

La composition du réactif à l'argent est la suivante :

| | | | |
|---|---|---|---|
| A | Carbonate de Na | 0,24 M | (SIGMA ref. S 4132) |
| B1 | Nitrate d'Ammonium | 13 mM | (SIGMA ref. A 9642) |
| B2 | Nitrate d'Argent | 6 mM | (BDH ref. 303873N) |
| B3 | Ac. Dodecatunstosilicique | 1,5 mM | (BDH ref. 305453R) |
| B4 | Formaldéhyde 37% | 0,6 ul/ml | (SIGMA ref. F 1635) |

Le réactif à l'argent est préparé en mélangeant successivement et sous agitation les solutions B1 - B2 - B3 - B4. Le mélange obtenu est additionné 1:1 à la solution A et le réactif ainsi préparé est utilisé immédiatement.

Après le traitement au réactif à l'argent, les lames sont lavées à l'H₂O distillée pendant 15 minutes, puis dans l'acide acétique 1% (v/v) 2 fois 15 minutes et enfin colorées pendant 1 minute en Pyronin Y (SIGMA ref. P 7017) 1% en H₂O, rincées pendant quelques secondes à l'eau distillée, séchées à l'air chaud et montées en DPX.

Bien que la description qui précède ait essentiellement fait état du tôle joué par la sonde b.c.1.2. pour la détermination du sexe des embryons de bovins, le contrôle de la présence du chromosome Y dans une population de spermatozoïdes et la séparation de cette dernière en deux groupes comprenant respectivement le chromosome Y et le chromosome X en vue de leur utilisation pour l'insémination artificielle, l'on comprendra aisément que ce rôle puisse s'étendre à d'autres animaux dans la mesure où ces derniers présentent des séquences d'ADN spécifique du sexe mâle similaires à celles desdits embryons de bovins; de même, le rôle de la sonde b.c.1.2. tel que décrit dans ce qui précède, est uniquement donné à titre d'exemple non limitatif et peut s'étendre à la recherche d'autres séquences spécifiques du sexe mâle.

## Revendications (Revendications pour l'(les) Etat(s) contractant(s) suivant(s): BE, CH, DE, FR, GB, IT, LI, LU, NL, SE)

1. Sonde moléculaire d'ADN spécifique du génome mâle des ruminants, notamment de la sous-famille des bovinés, et en particulier du genre Bos, laquelle sonde est caractérisée en ce qu'elle comprend un segment d'acide nucléique choisi dans le groupe constitué par :
a) un fragment d'acide nucléique de 49 paires de bases dont la séquence 5' - 3' est la suivante : 
5'                                                                                    3'
ATCAGTGCAGGGACCGAGATGTGCTCCAAGGAGTGTTTATCGGCTGCTT,
b) un fragment d'acide nucléique comprenant au moins 11 nucléotides consécutifs de ladite séquence,
c) un fragment d'acide nucléique dont la séquence présente au moins 70% d'homologie avec celle des segments a) et b) ci-dessus,
d) un fragment d'acide nucléique dont la séquence est complémentaire de celle de l'un des segments a), b) ou c) ci-dessus,
à condition que ledit fragment d'acide nucléique présente un profil d'hybridation spécifique avec l'ADN génomique mâle des ruminants digéré par EcoRI, qui fait apparaître la présence d'une bande de l'ordre de 7 kb.

2. Sonde moléculaire selon la Revendication 1, caractérisée en ce qu'elle est marquée avec un ou plusieurs radio-isotopes.

3. Sonde moléculaire selon la Revendication 1, caractérisée en ce qu'elle est marquée avec un marqueur non radioactif.

4. Procédé de préparation d'une sonde moléculaire d'ADN spécifique du génome mâle de ruminants, lequel procédé est caractérisé en ce qu'il comprend une étape au cours de laquelle on synthétise un fragment d'acide nucléique selon la Revendication 1.

5. Procédé selon la Revendication 4, caractérisé en ce que ledit fragment d'acide nucléique est synthétisé par la méthode de phosphoroamidite en phase solide.

6. Réactifs de diagnostic permettant la détection de séquences spécifiques du chromosome Y bovin, caractérisés en ce qu'ils comprennent un fragment d'acide nucléique selon l'une quelconque des Revendications 1 à 3.

7. Application des réactifs de diagnostic selon la Revendication 6, à la détermination *in vitro* du sexe d'embryons ou de foetus de ruminants.

8. Application des réactifs de diagnostic selon la Revendication 6, à la détection de spermatozoïdes portant le chromosome Y.

9. Procédé de contrôle direct et immédiat de la proportion de spermatozoïdes portant le chromosome Y dans une population de spermatozoïdes de ruminants, notamment de la sous-famille des bovinés, en particulier du genre Bos, caractérisé en ce que les spermatozoïdes de ladite population sont mis en contact avec une sonde d'ADN, selon l'une quelconque des Revendications 1 à 3, marquée d'une manière convenable, pour réaliser le marquage desdits spermatozoïdes par hybridation avec ladite sonde, puis les spermatozoïdes hybridés sont révélés par une méthode appropriée.

10. Procédé de contrôle selon la Revendication 9, caractérisé en ce que l'hybridation de la sonde est réalisée directement in situ, sans extraction préalable de l'ADN des spermatozoïdes.

11. Procédé de contrôle selon la Revendication 9, caractérisé en ce que l'ADN est isolé à partir des spermatozoïdes préalablement à l'hybridation avec la sonde.

12. Procédé de contrôle selon la Revendication 10, caractérisé en ce que les spermatozoïdes sont soumis préalablement à l'étape d'hybridation, à un processus de décondensation chimique qui comprend essentiellement trois étapes, à savoir : un choc hypotonique, une digestion protéolytique et une réduction des ponts disulfures.

13. Procédé de tri des spermatozoïdes porteurs du chromosome X et des spermatozoïdes porteurs du chromosome Y, dans une population de spermatozoïdes de ruminants, notamment de la sous-famille des bovinés, en particulier du genre Bos, caractérisé par les étapes suivantes :
a) marquage de l'ADN contenu dans les spermatozoïdes par un colorant vital spécifique de l'ADN ;
b) tri par cytofluorométrie en deux fractions de spermatozoïdes X et Y en fonction de la quantité d'ADN qu'ils contiennent ;
c) contrôle de la qualité du tri opéré au cours de la 2ème étape ci-dessus, en mettant en oeuvre le procédé de contrôle de la proportion de spermatozoïdes porteurs du chromosome Y selon l'une quelconque des Revendications 9 à 12 ;
d) immunisation de mammifères rongeurs (souris ou rats) par les fractions contrôlées, éventuellement soumises à un nouveau tri à la suite du contrôle, en vue de la production d'anticorps monoclonaux ou polyclonaux ;
e) contrôle de la spécificité desdits anticorps par des tests d'agglutination, de spermatotoxicité, d'immunofluorescence indirecte, de cytométrie de flux pour l'obtention de réactifs immunologiques spécifiques reconnaissant des déterminants antigéniques contrôlés par les chromosomes X ou Y et exprimés à la surface des spermatozoïdes, aptes à être utilisés pour la séparation de spermatozoïdes vivants porteurs du chromosome X ou du chromosome Y ;
d) séparation des fractions de spermatozoïdes porteurs respectivement du chromosome X et du chromosome Y, dans une population de spermatozoïdes, à l'aide desdits réactifs immunochimiques.

14. Procédé de tri selon la Revendication 13, caractérisé en ce que l'immunisation de mammifères rongeurs qui constitue la 4ème étape du procédé de tri est suivie de la collecte des anticorps polyclonaux résultant de ladite immunisation et de l'absorption de ceux-ci sur des spermatozoïdes de sexe opposé à celui ayant servi à l'immunisation, la spécificité de ces anticorps étant contrôlée sur des spermatozoïdes du sexe recherché, et lesdits anticorps étant ensuite éventuellement purifiés.

15. Procédé de tri selon la Revendication 13, caractérisé en ce que les spermatozoïdes X et Y séparés par cytofluorométrie sont utilisés pour préparer des anticorps monoclonaux spécifiques des chromosomes X et Y.

## Revendications (Revendications pour l'(les) Etat(s) contractant(s) suivant(s): AT, ES, GR)

1. Procédé de préparation d'une sonde moléculaire d'ADN spécifique du génome mâle de ruminants, lequel procédé est caractérisé en ce qu'il comprend une étape au cours de laquelle on synthétise un fragment d'acide nucléique choisi dans le groupe constitué par :
a) un fragment d'acide nucléique de 49 paires de bases dont la séquence 5' - 3' est la suivante : 
5'                                                                                    3'
ATCAGTGCAGGGACCGAGATGTGCTCCAAGGAGTGTTTATCGGCTGCTT,
b) un fragment d'acide nucléique comprenant au moins 11 nucléotides consécutifs de ladite séquence,
c) un fragment d'acide nucléique dont la séquence présente au moins 70% d'homologie avec celle des segments a) et b) ci-dessus,
d) un fragment d'acide nucléique dont la séquence est complémentaire de celle de l'un des segments a), b) ou c) ci-dessus,
à condition que ledit fragment d'acide nucléique présente un profil d'hybridation spécifique avec l'ADN génomique mâle des ruminants digéré par EcoRI, qui fait apparaître la présence d'une bande de l'ordre de 7 kb.

2. Procédé selon la Revendication 1, caractérisée en ce que le fragment d'acide nucléique synthétisé est marqué avec un ou plusieurs radio-isotopes.

3. Procédé selon la Revendication 1, caractérisée en ce que le fragment d'acide nucléique synthétisé est marqué avec un marqueur non radioactif.

4. Procédé selon la Revendication 1, caractérisé en ce que ledit fragment d'acide nucléique est synthétisé par la méthode de phosphoroamidite en phase solide.

5. Application d'une sonde moléculaire obtenue par le procédé selon l'une quelconque des Revendications 1 à 4, à la détermination *in vitro* du sexe d'embryons ou de foetus de ruminants.

6. Application d'une sonde moléculaire obtenue par le procédé selon l'une quelconque des Revendications 1 à 4, à la détection de spermatozoïdes portant le chromosome Y.

7. Procédé de contrôle direct et immédiat de la proportion de spermatozoïdes portant le chromosome Y dans une population de spermatozoïdes de ruminants, notamment de la sous-famille des bovinés, en particulier du genre Bos, caractérisé en ce que les spermatozoïdes de ladite population sont mis en contact avec une sonde d'ADN, obtenue par le procédé selon l'une quelconque des Revendications 1 à 4, marquée d'une manière convenable pour réaliser le marquage desdits spermatozoïdes par hybridation avec ladite sonde, puis les spermatozoïdes hybridés sont révélés par une méthode appropriée.

8. Procédé de contrôle selon la Revendication 7, caractérisé en ce que l'hybridation de la sonde est réalisée directement in situ, sans extraction préalable de l'ADN des spermatozoïdes.

9. Procédé de contrôle selon la Revendication 7, caractérisé en ce que l'ADN est isolé à partir des spermatozoïdes préalablement à l'hybridation avec la sonde.

10. Procédé de contrôle selon la Revendication 8, caractérisé en ce que les spermatozoïdes sont soumis préalablement à l'étape d'hybridation, à un processus de décondensation chimique qui comprend essentiellement trois étapes, à savoir : un choc hypotonique, une digestion protéolytique et une réduction des ponts disulfures.

11. Procédé de tri des spermatozoïdes porteurs du chromosome X et des spermatozoïdes porteurs du chromosome Y, dans une population de spermatozoïdes de ruminants, notamment de la sous-famille des bovinés, en particulier du genre Bos, caractérisé par les étapes suivantes :
a) marquage de l'ADN contenu dans les spermatozoïdes par un colorant vital spécifique de l'ADN ;
b) tri par cytofluorométrie en deux fractions de spermatozoïdes X et Y en fonction de la quantité d'ADN qu'ils contiennent ;
c) contrôle de la qualité du tri opéré au cours de la 2ème étape ci-dessus, en mettant en oeuvre le procédé de contrôle de la proportion de spermatozoïdes porteurs du chromosome Y selon l'une quelconque des Revendications 7 à 10 ;
d) immunisation de mammifères rongeurs (souris ou rats) par les fractions contrôlées, éventuellement soumises à un nouveau tri à la suite du contrôle, en vue de la production d'anticorps monoclonaux ou polyclonaux ;
e) contrôle de la spécificité desdits anticorps par des tests d'agglutination, de spermatotoxicité, d'immunofluorescence indirecte, de cytométrie de flux pour l'obtention de réactifs immunologiques spécifiques reconnaissant des déterminants antigéniques contrôlés par les chromosomes X ou Y et exprimés à la surface des spermatozoïdes, aptes à être utilisés pour la séparation de spermatozoïdes vivants porteurs du chromosome X ou du chromosome Y ;
d) séparation des fractions de spermatozoïdes porteurs respectivement du chromosome X et du chromosome Y, dans une population de spermatozoïdes, à l'aide desdits réactifs immunochimiques.

12. Procédé de tri selon la Revendication 12, caractérisé en ce que l'immunisation de mammifères rongeurs qui constitue la 4ème étape du procédé de tri est suivie de la collecte des anticorps polyclonaux résultant de ladite immunisation et de l'absorption de ceux-ci sur des spermatozoïdes de sexe opposé à celui ayant servi à l'immunisation, la spécificité de ces anticorps étant contrôlée sur des spermatozoïdes du sexe recherché, et lesdits anticorps étant ensuite éventuellement purifiés.

13. Procédé de tri selon la Revendication 12, caractérisé en ce que les spermatozoïdes X et Y séparés par cytofluorométrie sont utilisés pour préparer des anticorps monoclonaux spécifiques des chromosomes X et Y.

## Claims (Claims for the following Contracting State(s): BE, CH, DE, FR, GB, IT, LI, LU, NL, SE)

1. DNA molecular probe specific for the male genome of ruminants, in particular of the subfamily of bovines and especially of the genus Bos, which probe is characterised in that it comprises a nucleic acid segment chosen from the group consisting of:
a) a 49-base pair nucleic acid fragment whose 5' - 3' sequence is as follows: 
5′                                                    3′
ATCAGTGCAGGGACCGAGATGTGCTCCAAGGAGTGTTTATCGGCTGCTT,
b) a nucleic acid fragment comprising at least 11 consecutive nucleotides of the said sequence,
c) a nucleic acid fragment whose sequence displays an at least 70% homology with that of the above segments a) and b),
d) a nucleic acid fragment whose sequence is complementary to that of one of the above segments a), b) or c),
on condition that the said nucleic acid fragment displays a specific hybridisation profile with EcoRI-digested male genomic DNA of ruminants which reveals the presence of a band of the order of 7 kb.

2. Molecular probe according to Claim 1, characterised in that it is labelled with one or more radioisotopes.

3. Molecular probe according to Claim 1, characterised in that it is labelled with a non-radioactive label.

4. Method for preparing a DNA molecular probe specific for the male genome of ruminants, which process is characterised in that it comprises a step during which a nucleic acid fragment according to Claim 1 is synthesised.

5. Method according to Claim 4, characterised in that the said nucleic acid fragment is synthesized by the solid-phase phosphoramidite method.

6. Diagnostic reagents that enable specific sequences of the bovine Y chromosome to be detected, characterised in that they comprise a nucleic acid fragment according to any one of Claims 1 to 3.

7. Application of the diagnostic reagents according to Claim 6 to the *in vitro* determination of the sex of ruminant embryos or fetuses.

8. Application of the diagnostic reagents according to Claim 6 to the detection of spermatozoa carrying the Y chromosome.

9. Method for the direct and immediate monitoring of the proportion of spermatozoa carrying the Y chromosome in a population of spermatozoa of ruminants, in particular of the subfamily of bovines and especially of the genus Bos, characterised in that the spermatozoa of the said population are brought into contact with a suitably labelled DNA probe according to any one of Claims 1 to 3 in order to effect labelling of the said spermatozoa by hybridisation with the said probe, and the hybridised spermatozoa are then visualised by a suitable method.

10. Monitoring method according to Claim 9, characterised in that the hybridisation of the probe is carried out directly in situ, without prior extraction of the DNA from the spermatozoa.

11. Monitoring method according to Claim 9, characterised in that the DNA is isolated from the spermatozoa prior to the hybridisation with the probe.

12. Monitoring method according to Claim 10, characterised in that the spermatozoa are subjected, prior to the hybridisation step, to a process of chemical decondensation which essentially comprises three steps, namely: a hypotonic shock, a proteolytic digestion and a reduction of the disulphide bridges.

13. Method for sorting the spermatozoa carrying the X chromosome and the spermatozoa carrying the Y chromosome in a population of spermatozoa of ruminants, in particular of the subfamily of bovines and especially of the genus Bos, characterised by the following steps:
a) labelling of the DNA contained in the spermatozoa with a vital stain specific for DNA;
b) sorting by cytofluorometry into two fractions of spermatozoa X and Y, in accordance with the quantity of DNA they contain;
c) monitoring of the quality of the sorting carried out in the 2nd step above, by implementing the method for monitoring the proportion of spermatozoa carrying the Y chromosome according to any one of Claims 9 to 12;
d) immunisation of rodent mammals (mice or rats) with the monitored fractions, where appropriate subjected to a fresh sorting following the monitoring, for the purpose of producing monoclonal or polyclonal antibodies;
e) monitoring of the specificity of the said antibodies by agglutination, spermatotoxicity, indirect immunofluorescence or flow cytometry tests in order to obtain specific immunological reagents that recognise antigenic determinants controlled by the X or Y chromosomes and expressed at the surface of the spermatozoa, and which are capable of being used for the separation of living spermatozoa carrying the X chromosome or the Y chromosome;
f) separation of the fractions of spermatozoa carrying the X chromosome and the Y chromosome, respectively, in a population of spermatozoa, using the said immunochemical reagents.

14. Sorting method according to Claim 13, characterised in that the immunisation of rodent mammals which constitutes the 4th step of the sorting method is followed by collection of the polyconal antibodies resulting from the said immunisation and absorption of these antibodies on spermatozoa of the sex opposite to that used for the immunisation, the specificity of these antibodies being monitored on spermatozoa of the sex which is sought and the said antibodies then being purified where appropriate.

15. Sorting method according to Claim 13, characterised in that the X and Y spermatozoa separated by cytofluorometry are used to prepare monoclonal antibodies specific for the X and Y chromosomes.

## Claims (Claims for the following Contracting State(s): AT, ES, GR)

1. Method for preparing a DNA molecular probe specific for the male genome of ruminants, which method is characterised in that it comprises a step during which there is synthesised a nucleic acid fragment chosen from the group consisting of:
a) a 49-base pair nucleic acid fragment whose 5' - 3' sequence is as follows: 
5′ 3′
ATCAGTGCAGGGACCGAGATGTGCTCCAAGGAGTGTTTATCGGCTGCTT,
b) a nucleic acid fragment comprising at least 11 consecutive nucleotides of the said sequence,
c) a nucleic acid fragment whose sequence displays an at least 70% homology with that of the above segments a) and b),
d) a nucleic acid fragment whose sequence is complementary to that of one of the above segments a), b) or c),
on condition that the said nucleic acid fragment displays a specific hybridisation profile with EcoRI-digested male genomic DNA of ruminants which reveals the presence of a band of the order of 7 kb.

2. Method according to Claim 1, characterised in that the nucleic acid fragment synthesized is labelled with one or more radioisotopes.

3. Method according to Claim 1, characterised in that the nucleic acid fragment synthesised is labelled with a non-radioactive label.

4. Method according to Claim 1, characterized in that the said nucleic acid fragment is synthesized by the solid-phase phosphoramidite method.

5. Application of a molecular probe obtained by the method according to any one of Claims 1 to 4 to the *in vitro* determination of the sex of embryos or fetuses of ruminants.

6. Application of a molecular probe obtained by the method according to any one of Claims 1 to 4 to the detection of spermatozoa carrying the Y chromosome.

7. Method for the direct and immediate monitoring of the proportion of spermatozoa carrying the Y chromosome in a population of spermatozoa of ruminants, in particular of the subfamily of bovines and especially of the genus Bos, characterised in that the spermatozoa of the said population are brought into contact with a suitably labelled DNA probe obtained by the method according to any one of Claims 1 to 4 in order to effect labelling of the said spermatozoa by hybridisation with the said probe, and the hybridised spermatozoa are then visualised by a suitable method.

8. Monitoring method according to Claim 7, characterised in that the hybridisation of the probe is carried out directly in situ, without prior extraction of the DNA from the spermatozoa.

9. Monitoring method according to Claim 7, characterised in that the DNA is isolated from the spermatozoa prior to the hybridisation with the probe.

10. Monitoring method according to Claim 8, characterised in that the spermatozoa are subjected, prior to the hybridisation step, to a process of chemical decondensation which essentially comprises three steps, namely: a hypotonic shock, a proteolytic digestion and a reduction of the disulphide bridges.

11. Method for sorting the spermatozoa carrying the X chromosome and the spermatozoa carrying the Y chromosome in a population of spermatozoa of ruminants, in particular of the subfamily of bovines and especially of the genus Bos, characterised by the following steps:
a) labelling of the DNA contained in the spermatozoa with a vital stain specific for DNA;
b) sorting by cytofluorometry into two fractions of spermatozoa X and Y, in accordance with the quantity of DNA they contain;
c) monitoring of the quality of the sorting carried out in the 2nd step above, by implementing the method for monitoring the proportion of spermatozoa carrying the Y chromosome according to any one of Claims 7 to 10;
d) immunisation of rodent mammals (mice or rats) with the monitored fractions, where appropriate subjected to a fresh sorting following the monitoring, for the purpose of producing monoclonal or polyconal antibodies;
e) monitoring of the specificity of the said antibodies by agglutination, spermatotoxicity, indirect immunofluorescence or flow cytometry tests in order to obtain specific immunological reagents that recognise antigenic determinants controlled by the X or Y chromosomes and expressed at the surface of the spermatozoa, and which are capable of being used for the separation of living spermatozoa carrying the X chromosome or the Y chromosome;
f) separation of the fractions of spermatozoa carrying the X chromosome and the Y chromosome, respectively, in a population of spermatozoa, using the said immunochemical reagents.

12. Sorting method according to Claim 11, characterised in that the immunisation of rodent mammals which constitutes the 4th step of the sorting method is followed by collection of the polyconal antibodies resulting from the said immunisation and absorption of these antibodies on spermatozoa of the sex opposite to that used for the immunisation, the specificity of these antibodies being monitored on spermatozoa of the sex which is sought and the said antibodies then being purified where appropriate.

13. Sorting method according to Claim 11, characterised in that the X and Y spermatozoa separated by cytofluorometry are used to prepare monoclonal antibodies specific for the X and Y chromosomes.

## Patentansprüche (Patentansprüche für folgende(n) Vertragsstaat(en): BE, CH, DE, FR, GB, IT, LI, LU, NL, SE)

1. Molekulare DNA-Sonde, spezifisch für das männliche Genom von Wiederkäuern, insbesondere der Unterfamilie der Rinder, und insbesondere der Gattung Bos, dadurch **gekennzeichnet,** daß die Sonde ein Nukleinsäuresegment, ausgewählt aus der Gruppe:
(a) Nukleinsäurefragment aus 49 Basenpaaren, deren 5'-3'-Sequenz wie folgt lautet: 
5'                                                                                                    3'
ATCAGTGCAGGGACCGAGATGTGCTCCAAGGAGTGTTTATCGGCTGCTT,
(b) Nukleinsäurefragment, umfassend mindestens 11 aufeinanderfolgende Nukleotide dieser Sequenz,
(c) Nukleinsäurefragment, dessen Sequenz mindestens 70 % Homologie mit der der obigen Segmente (a) und (b) zeigt,
(d) Nukleinsäurefragment, dessen Sequenz der eines der obigen Segmente (a), (b) oder (c) komplementär ist, umfaßt, mit der Maßgabe, daß das Nukleinsäurefragment ein für die männliche genomische DNA von Wiederkäuern, die durch EcoRI, das zum Erscheinen einer Bande in der Größenordnung von 7 kb führt, abgebaut wurde, spezifisches Hybridisierungsprofil zeigt.

2. Molekulare Sonde nach Anspruch 1, dadurch **gekennzeichnet,** daß sie mit einem oder mehreren Radioisotopen markiert ist.

3. Molekulare Sonde nach Anspruch 1, dadurch **gekennzeichnet,** daß sie mit einem nicht-radioaktiven Marker markiert ist.

4. Verfahren zur Herstellung einer molekularen DNA-Sonde, die für das männliche Genom von Wiederkäuern spezifisch ist, dadurch **gekennzeichnet,** daß es eine Stufe umfaßt, im Verlauf derer man ein Nukleinsäurefragment nach Anspruch 1 synthetisiert.

5. Verfahren nach Anspruch 4, dadurch **gekennzeichnet,** daß das Nukleinsäurefragment nach dem Phosphoramiditverfahren an fester Phase synthetisiert wird.

6. Diagnostische Reagenzien zur Detektion von Sequenzen, die für das Y-Chromosom des Rinds spezifisch sind, dadurch **gekennzeichnet,** daß sie ein Nukleinsäurefragment nach einem der Ansprüche 1 bis 3 umfassen.

7. Verwendung der diagnostischen Reagenzien nach Anspruch 6 zur in-vitro-Bestimmung des Geschlechts eines Embryos oder Fötus von Wiederkäuern.

8. Verwendung der diagnostischen Reagenzien nach Anspruch 6 zur Detektion der Spermien, die das Y-Chromosom tragen.

9. Verfahren zur direkten und unmittelbaren Kontrolle des Anteils der Spermien, die das Y-Chromosom tragen, in einer Spermienpopulation von Wiederkäuern, insbesondere der Unterfamilie der Rinder, insbesondere der Gattung Bos, dadurch **gekennzeichnet,** daß die Spermien dieser Population mit einer DNA-Sonde nach einem der Ansprüche 1 bis 3, die geeigneterweise markiert ist, in Kontakt gebracht werden, um die Spermien durch Hybridisierung mit der Sonde zu markieren, und danach die hybridisierten Spermien nach einem geeigneten Verfahren detektiert werden.

10. Verfahren zur Kontrolle nach Anspruch 9, dadurch **gekennzeichnet,** daß die Hybridisierung der Sonde direkt in situ ohne vorherige Extraktion der DNA der Spermien durchgeführt wird.

11. Verfahren zur Kontrolle nach Anspruch 9, dadurch **gekennzeichnet,** daß die DNA ausgehend von zuvor mit der Sonde hybridisierten Spermien isoliert wird.

12. Verfahren zur Kontrolle nach Anspruch 10, dadurch **gekennzeichnet,** daß die Spermien bei dem Hybridisierungsschritt zuerst einem chemischen Dekondensationsverfahren unterworfen werden, das im wesentlichen drei Schritte umfaßt, nämlich einen hypotonen Schock, einen proteolytischen Abbau und eine Reduktion der Disulfidbrücken.

13. Verfahren zur Aussonderung von Spermien, die das X-Chromosom tragen, und von Spermien, die das Y-Chromosom tragen, in einer Spermienpopulation von Wiederkäuern, insbesondere der Unterfamilie der Rinder, insbesondere der Gattung Bos, **gekennzeichnet** durch die folgenden Stufen:
(a) Markierung der bekannten DNA in den Spermien durch einen spezifischen Vitalfarbstoff für DNA;
(b) cytofluorometrische Auftrennung in zwei Spermienfraktionen X und Y als Funktion der DNA-Menge, die sie enthalten;
(c) Kontrolle der Qualität der im Verlauf der obigen zweiten Stufe durchgeführten Auftrennung, dadurch, daß man das Kontrollverfahren des Teils der Spermien, die das Y-Chromosom tragen, nach einem der Ansprüche 9 bis 12 durchführt;
(d) Immunisierung von Nagetieren [Säugetieren (Mäuse oder Ratten)] durch die kontrollierten Fraktionen, die gegebenenfalls einer erneuten Auftrennung als Folge der Kontrolle unterworfen worden sind, um monoklonale oder polyklonale Antikörper zu erzeugen;
(e) Kontrolle der Spezifität der Antikörper durch Agglutinationstests, Tests auf Spermatotoxizität, Tests auf indirekte Immunfluoreszenz, Durchflußcytometrie, um spezifische immunologische Reagenzien zu erhalten, die die antigenen Determinanten, die von den Chromosomen X oder Y kontrolliert werden und an der Oberfläche der Spermien exprimiert werden, zu erhalten, welche zur Verwendung zur Trennung von lebenden Spermien, die das Chromosom X oder das Chromosom Y tragen, geeignet sind;
(f) Abtrennung der Spermienfraktionen, die jeweils das Chromosom X bzw. das Chromosom Y tragen, aus einer Spermienpopulation mit Hilfe der immunchemischen Reagenzien.

14. Verfahren zur Aussonderung nach Anspruch 13, dadurch **gekennzeichnet,** daß sich an die Immunisierung der Nagetiere, welche die vierte Stufe des Aussonderungsverfahrens darstellt, die Gewinnung der polyklonalen Antikörper, die sich aus der Immunisierung ergeben, und ihre Absorption auf den Spermien des Geschlechts, das dem, das zur Immunisierung gedient hat, entgegengesetzt ist, anschließt, wobei die Spezifität dieser Antikörper auf den Spermien des gesuchten Geschlechts kontrolliert wird und die Antikörper anschließend gegebenenfalls gereinigt werden.

15. Verfahren zur Aussonderung nach Anspruch 13, dadurch **gekennzeichnet,** daß die X- und Y-Spermien, die durch Cytofluorometrie getrennt worden sind, zur Herstellung spezifischer monoklonaler Antikörper der Chromosomen X und Y verwendet werden.

## Patentansprüche (Patentansprüche für folgende(n) Vertragsstaat(en): AT, ES, GR)

1. Verfahren zur Herstellung einer molekularen DNA-Sonde, die für das männliche Genom von Wiederkäuern spezifisch ist, dadurch **gekennzeichnet,** daß es eine Stufe umfaßt, im Verlauf derer man ein Nukleinsäurefragment synthetisiert, ausgewählt aus der Gruppe, bestehend aus:
(a) Nukleinsäurefragment aus 49 Basenpaaren, deren 5'-3'-Sequenz wie folgt lautet: 
5'                                                                                                    3'
ATCAGTGCAGGGACCGAGATGTGCTCCAAGGAGTGTTTATCGGCTGCTT,
(b) Nukleinsäurefragment, umfassend mindestens 11 aufeinanderfolgende Nukleotide dieser Sequenz,
(c) Nukleinsäurefragment, dessen Sequenz mindestens 70 % Homologie mit der der obigen Segmente (a) und (b) zeigt,
(d) Nukleinsäurefragment, dessen Sequenz der eines der obigen Segmente (a), (b) oder (c) komplementär ist, umfaßt, mit der Maßgabe, daß das Nukleinsäurefragment ein für die männliche genomische DNA von Wiederkäuern, die durch EcoRI, das zum Erscheinen einer Bande in der Größenordnung von 7 kb führt, abgebaut wurde, spezifisches Hybridisierungsprofil zeigt.

2. Verfahren nach Anspruch 1, dadurch **gekennzeichnet,** daß das synthetisierte Nukleinsäurefragment mit einem oder mehreren Radioisotopen markiert ist.

3. Verfahren nach Anspruch 1, dadurch **gekennzeichnet,** daß das synthetisierte Nukleinsäurefragment mit einem nicht-radioaktiven Marker markiert ist.

4. Verfahren nach Anspruch 1, dadurch **gekennzeichnet,** daß das Nukleinsäurefragment nach dem Phosphoramiditverfahren an fester Phase synthetisiert wird.

5. Verwendung einer nach dem Verfahren nach einem der Ansprüche 1 bis 4 erhaltenen molekularen Sonde zur in-vitro-Bestimmung des Geschlechts eines Embryos oder Fötus von Wiederkäuern.

6. Verwendung einer nach dem Verfahren nach einem der Ansprüche 1 bis 4 erhaltenen molekularen Sonde zur Detektion der Spermien, die das Y-Chromosom tragen.

7. Verfahren zur direkten und unmittelbaren Kontrolle des Anteils der Spermien, die das Y-Chromosom tragen, in einer Spermienpopulation von Wiederkäuern, insbesondere der Unterfamilie der Rinder, insbesondere der Gattung Bos, dadurch **gekennzeichnet,** daß die Spermien dieser Population mit einer DNA-Sonde, erhalten nach dem Verfahren nach einem der Ansprüche 1 bis 4, die geeigneterweise markiert ist, in Kontakt gebracht werden, um die Spermien durch Hybridisierung mit der Sonde zu markieren, und danach die hybridisierten Spermien nach einem geeigneten Verfahren detektiert werden.

8. Verfahren zur Kontrolle nach Anspruch 7, dadurch **gekennzeichnet,** daß die Hybridisierung der Sonde direkt in situ ohne vorherige Extraktion der DNA der Spermien durchgeführt wird.

9. Verfahren zur Kontrolle nach Anspruch 7, dadurch **gekennzeichnet,** daß die DNA ausgehend von zuvor mit der Sonde hybridisierten Spermien isoliert wird.

10. Verfahren zur Kontrolle nach Anspruch 8, dadurch **gekennzeichnet,** daß die Spermien bei dem Hybridisierungsschritt zuerst einem chemischen Dekondensationsverfahren unterworfen werden, das im wesentlichen drei Schritte umfaßt, nämlich einen hypotonen Schock, einen proteolytischen Abbau und eine Reduktion der Disulfidbrücken.

11. Verfahren zur Aussonderung von Spermien, die das X-Chromosom tragen, und von Spermien, die das Y-Chromosom tragen, in einer Spermienpopulation von Wiederkäuern, insbesondere der Unterfamilie der Rinder, insbesondere der Gattung Bos, **gekennzeichnet** durch die folgenden Stufen:
(a) Markierung der bekannten DNA in den Spermien durch einen spezifischen Vitalfarbstoff für DNA;
(b) cytofluorometrische Auftrennung in zwei Spermienfraktionen X und Y als Funktion der DNA-Menge, die sie enthalten;
(c) Kontrolle der Qualität der im Verlauf der obigen zweiten Stufe durchgeführten Auftrennung, dadurch, daß man das Kontrollverfahren des Teils der Spermien, die das Y-Chromosom tragen, nach einem der Ansprüche 7 bis 10 durchführt;
(d) Immunisierung von Nagetieren [Säugetieren (Mäuse oder Ratten)] durch die kontrollierten Fraktionen, die gegebenenfalls einer erneuten Auftrennung als Folge der Kontrolle unterworfen worden sind, um monoklonale oder polyklonale Antikörper zu erzeugen;
(e) Kontrolle der Spezifität der Antikörper durch Agglutinationstests, Tests auf Spermatotoxizität, Tests auf indirekte Immunfluoreszenz, Durchflußcytometrie, um spezifische immunologische Reagenzien zu erhalten, die die antigenen Determinanten, die von den Chromosomen X oder Y kontrolliert werden und an der Oberfläche der Spermien exprimiert werden, zu erhalten, welche zur Verwendung zur Trennung von lebenden Spermien, die das Chromosom X oder das Chromosom Y tragen, geeignet sind;
(f) Abtrennung der Spermienfraktionen, die jeweils das Chromosom X bzw. das Chromosom Y tragen, aus einer Spermienpopulation mit Hilfe der immunchemischen Reagenzien.

12. Verfahren zur Aussonderung nach Anspruch 11, dadurch **gekennzeichnet,** daß sich an die Immunisierung der Nagetiere, welche die vierte Stufe des Aussonderungsverfahrens darstellt, die Gewinnung der polyklonalen Antikörper, die sich aus der Immunisierung ergeben, und ihre Absorption auf den Spermien des Geschlechts, das dem, das zur Immunisierung gedient hat, entgegengesetzt ist, anschließt, wobei die Spezifität dieser Antikörper auf den Spermien des gesuchten Geschlechts kontrolliert wird und die Antikörper anschließend gegebenenfalls gereinigt werden.

13. Verfahren zur Aussonderung nach Anspruch 11, dadurch **gekennzeichnet,** daß die X- und Y-Spermien, die durch Cytofluorometrie getrennt worden sind, zur Herstellung spezifischer monoklonaler Antikörper der Chromosomen X und Y verwendet werden.
